# EUROPEAN PATENT SPECIFICATION

(11) **EP 2 649 184 B1**
(45) Date of publication and mention of the grant of the patent: **07.10.2015**
(21) Application number: 12795606.8
(22) Date of filing: 30.11.2012
(51) Int. Cl.: C12N 15/85, A01K 67/027, C12N 9/64, C07K 16/00

(54) **USE OF FERTILE TRANSGENIC ANIMALS FOR PRODUCING ANTIBODIES BEARING HUMAN VARIABLE REGIONS**
VERWENDUNG VON FRUCHTBAREN TRANSGENEN TIEREN ZUR HERSTELLUNG VON ANTIKÖRPERN MIT MENSCHLICHEN VARIABLEN REGIONEN
UTILISATION DES ANIMAUX TRANSGÉNIQUES FERTILES POUR LA PRODUCTION D'ANTICORPS PORTANT DES RÉGIONS VARIABLES HUMAINES

(30) Priority: 02.12.2011 US 201113310431; 21.12.2011 GB 201122047; 09.03.2012 US 201213416684
(43) Date of publication of application: 16.10.2013
(73) Proprietor: Kymab Limited, Cambridge CB22 3AT (GB)
(72) Inventor: FRIEDRICH, Glenn A, Cambridge CB22 3AT (GB); LEE, E-Chiang, Cambridge CB22 3AT (GB)
(74) Representative: Hollywood, Jane Constance
(86) International application number: PCT/GB2012/052956
(87) International publication number: WO 2013/079953

(56) References cited:
- WO-A1-02/066630
- WO-A1-2011/004192
- WO-A1-2012/141798
- WO-A1-2013/022782
- WO-A1-2013/096142
- WO-A1-2013/116609
- WO-A2-2008/076379
- LONBERG ET AL: "Fully human antibodies from transgenic mouse and phage display platforms", CURRENT OPINION IN IMMUNOLOGY, ELSEVIER, OXFORD, GB, vol. 20, no. 4, 1 August 2008 (2008-08-01) , pages 450-459, XP025771204, ISSN: 0952-7915, DOI: 10.1016/J.COI.2008.06.004 [retrieved on 2008-07-21]
- CECIL HAN ET AL: "Comprehensive analysis of reproductive ADAMs: relationship of ADAM4 and ADAM6 with an ADAM complex required for fertilization in mice", BIOLOGY OF REPRODUCTION, NEW YORK, NY [U.A.] : ACADEM. PRESS, US, vol. 80, no. 5, 1 May 2009 (2009-05-01), pages 1001-1008, XP002677427, ISSN: 0006-3363, DOI: 10.1095/BIOLREPROD.108.073700 [retrieved on 2009-01-07]
- M. R. MARCELLO ET AL: "Lack of Tyrosylprotein Sulfotransferase-2 Activity Results in Altered Sperm-Egg Interactions and Loss of ADAM3 and ADAM6 in Epididymal Sperm", JOURNAL OF BIOLOGICAL CHEMISTRY, vol. 286, no. 15, 21 February 2011 (2011-02-21), pages 13060-13070, XP55053699, ISSN: 0021-9258, DOI: 10.1074/jbc.M110.175463
- ESTHER ZURITA ET AL: "Genetic polymorphisms among C57BL/6 mouse inbred strains", TRANSGENIC RESEARCH, KLUWER ACADEMIC PUBLISHERS-PLENUM PUBLISHERS, NE, vol. 20, no. 3, 27 May 2010 (2010-05-27), pages 481-489, XP019900612, ISSN: 1573-9368, DOI: 10.1007/S11248-010-9403-8
- "Janeway's Immunobiology 7th edition", 2008, Garland Science, New York, London ISBN: 0815341237 page 158,
- ANONYMOUS: 'Mouse ES cell lines', [Online] Retrieved from the Internet: <URL:http://www.research.uci.edu/facilities -services/tmf/presentations/Mouse_ES_CellLi ne.pdf>
- RODRÍGUEZ C I ET AL: "High-efficiency deleter mice show that FLPe is an alternative to Cre-loxP.", NATURE GENETICS JUN 2000, vol. 25, no. 2, June 2000 (2000-06), pages 139-140, ISSN: 1061-4036

## Description

The present invention relates *inter alia* to the use of fertile mice and rats useful for producing antibodies bearing human variable regions, in which endogenous antibody chain expression has been inactivated.

### BACKGROUND

Antibody-generating non-human vertebrates such as mice and ratsthat comprise one or more transgenic antibody loci encoding variable regions are generally known in the art, and by way of example reference is made to WO2011004192, US7501552, US6673986, US6130364, WO2009/076464 and US6586251.

Using embryonic stem cell (EScell) technology, the art has provided non-human vertebrates, such as mice and rats, bearing transgenic antibody loci from which human or chimaeric antibodies can be generated *in vivo* following challenge with human antigen. Such antibodies usefully bear human variable regions in their heavy chains and optionally also in their light chains. In order to avoid complications of endogenous antibody heavy chain expression at the same time, the genomes of such vertebrates are typically engineered so that endogenous heavy chain expression is inactivated. Techniques for doing this involve the deletion of all or part of the endogenous heavy chain VDJ region simultanesously with the insertion of human VDJgene segments or in a separate step (eg, see WO2009076464 and WO2002066630). Such deletion entails the deletion of VH and D gene segments along with the intervening sequences. In doing so, the endogenous ADAM 6 genes are deleted.

The ADAM 6 gene encodes a protein belonging to the A disintegrin and metalloprotease (ADAM) family. ADAM family members are transmembrane glycoproteins that contain conserved multi-domains such as pro-domain, metalloprotease, disintegrin, cysteine-rich, epidermal growth factor (EGF)-like, transmembrane, and cytoplasmic tail domains. TheADAM family has been shown to be involved in cell adhesion [1-5] in various biological progress.

In mouse, there are two copies of ADAM 6 (ADAM 6a, ADAM 6b) located between the VH and D gene segments in the IgH locus of chromosome 12 (in the intervening region between mouse V_{H}5-1 and D1-1 gene segments). These two adjacent intronlessADAM 6 genes have 95% nucleotide sequence identity and 90% amino acid identity. In human and rat, there is only one ADAM6 gene. Expression pattern analysis of mouse ADAM 6 shows that it is exclusively expressed in testis [6]. Although ADAM 6 transcripts can be detected in lymphocytes, it is restricted to the nucleus, suggesting that the transcription of ADAM 6 gene in particular is due to transcriptional read-through from the D region rather than active messenger RNA production [7].

Mature ADAM 6 protein is located on the acrosome and the posterior regions of sperm head. Notably, ADAM 6 forms a complex with ADAM 2 and ADAM3, which is required for fertilization in mice [8]. Reference [9] implicatesADAM6 in amodel where thisprotein interactswith ADAM 3 after ADAM 6 is sulphated by TPST2, sulphation of ADAM 6 being critical for stability and/or complex formation involving ADAM6 and ADAM3, and thus ADAM 6 and ADAM 3 are lost from *Tpst2*-null sperm. The study observes that *Tpst2*-deficient mice have male infertility, sperm mobility defects and possible abnormalities in sperm-egg membrane interactions.

Thus, the maintenance of ADAM 6 expression in sperm is crucial for fertility. Thus, it is thought that transgenic male mice and rats in which ADAM 6 genes have been deleted are not viably fertile. This hampers breeding of colonies and hampers the utility of such mice as transgenic antibody-generating platforms. It would be desirable to provide improved non-human transgenic antibody-generating vertebratesthat are fertile.

### References

[1] Primakoff P, Myles DG. The ADAM gene family: surface proteins with adhesion and protease activity. Trends Genet. 2000 Feb;16(2):83-7.
[2] Evans JP. Fertilin beta and other ADAMs as integrin ligands: insights into cell adhesion and fertilization. Bioessays. 2001 Jul;23(7):628-39.
[3] Primakoff P, Myles DG. Penetration, adhesion, and fusion in mammalian sperm-egg interaction. Science. 2002 Jun 21;296(5576):2183-5.
[4] Talbot P, Shur BD, Myles DG. Cell adhesion and fertilization: steps in oocyte transport, sperm-zona pellucida interactions, and sperm-egg fusion. Biol Reprod. 2003 Jan;68(1):1-9.
[5] Huovila AP et. al., Shedding light on ADAM metalloproteinases. Trends Biochem Sci. 2005 Jul;30(7):413-22.
[6] Choi I, et. al., Characterization and comparative genomic analysisof intronlessAdamswith testicular gene expression. Genomics. 2004 Apr;83(4):636-46.
[7] Featherstone K, Wood AL, Bowen AJ, Corcoran AE. The mouse immunoglobulin heavy chain V-D intergenic sequence contains insulators that may regulate ordered V(D)J recombination. J Biol Chem. 2010 M ar 26;285(13):9327-38. Epub 2010 Jan 25.
[8] Han C, et. al., Comprehensive analysis of reproductive ADAMs: relationship of ADAM4 and ADAM 6 with an ADAM complex required for fertilization in mice. Biol Reprod. 2009 May;80(5):1001-8. Epub 2009 Jan 7.
[9] Marcello et al, Lack of tyrosylprotein sulfotransferase-2 activity results in altered sperm-egg interactions and lossof ADAM3 and ADAM6 in epididymal sperm, J Biol Chem. 2011 Apr 15;286(15):13060-70. Epub 2011 Feb 21.

### SUMMARY OF THE INVENTION

To thisend, the present invention is defined in the claims.

Disclosed herein is:
A method of making a fertile non-human vertebrate (eg, a mouse) that is homozygous for a transgenic antibody heavy chain locus,
the mouse having a genome that
   (a) comprises each transgenic heavy chain locus on a respective copy of chromosome 12 (or equivalent chromosome for said vertebrate); and
   (b) is inactivated for endogenous antibody heavy chain expression;
      the method comprising the stepsof
   (c) constructing a transgenic mouse embryonic stem cell (ES cell) comprising a transgenic
      antibody heavy chain locus by inserting one or more human VH gene segments, one or more human D gene segments and one or more human JH gene segmentsinto DNA of achromosome 12 (or equivalent chromosome for said vertebrate) so that the human gene segments are operably connected upstream of a mouse or human endogenous heavy chain constant region (optionally Cmu and/or Cgamma);
   (d) simultaneously or separately from step (c), deleting all or part of the mouse endogenous heavy chain VDJregion of said chromosome 12 to inactivate endogenous-antibody heavy chain expression, wherein the deletion includes mouse ADAM6-encoding nucleotide sequence;
   (e) simultaneously or separately from step (c) or (d), inserting into the ES cell genome one or more ADAM6-encoding nucleotide sequences; and
   (f) developing the EScell into a fertile mouse or a progeny thereof whose genome is homorygous for said transgenic heavy chain locus and encodes ADAM 6, wherein all or part of the endogenous heavy chain VDJregion has been deleted from both chromosomes 12 in the genome; optionally wherein said fertile mouse or progeny is male.

Dislcosed herein is a method of making a fertile non-human vertebrate (eg, a mouse) that is homozygous for a transgenic antibody heavy chain locus,
the mouse having a genome that
(a) comprises each transgenic heavy chain locus on a respective copy of chromosome 12 (or equivalent chromosome for said vertebrate); and
(b) is inactivated for endogenous antibody heavy chain expression;
   the method comprising the steps of
(c) constructing atransgenic mouse embryonic stem cell (ES cell) comprising a transgenic antibody heavy chain locus by inserting one or more human VH gene segments, one or more human D gene segments and one or more human JH gene segments into DNA of a chromosome 12 so that the human gene segments are operably connected upstream of a mouse or human endogenous heavy chain constant region (optionally Cmu and/or Cgamma);
(d) simultaneously or separately from step (c), deleting all or part of the mouse endogenous heavy chain VDJregion of said chromosome 12 to inactivate endogenous antibody heavy chain expression, wherein the deletion includes mouse ADAM6-encoding nucleotide sequences;
(e) developing the ES cell into a child mouse or progeny thereof whose genome comprises a said transgenic heavy chain locus;
(f) deriving a second EScell from said mouse and inserting into the genome of said second ES cell one or more ADAM 6-encoding nucleotide sequences; and
(g) developing the second EScell into a fertile mouse or a progeny thereof whose genome is homozygous for said transgenic heavy chain locus and encodes ADAM 6, wherein all or part of the endogenous heavy chain VDJregion has been deleted from both chromosomes 12 in the genome; optionally wherein said fertile mouse or progeny is male.

Disclosed herein is a method of making a fertile non-human vertebrate (eg, a mouse) that is homozygous for a transgenic antibody heavy chain locus,
the mouse having a genome that
(a) comprises each transgenic heavy chain locus on a respective copy of chromosome 12 (or equivalent chromosome for said vertebrate); and
(b) is inactivated for endogenous antibody heavy chain expression;
   the method comprising the steps of
(c) constructing a transgenic mouse embryonic stem cell (ES cell) comprising a transgenic antibody heavy chain locus by inserting one or more human VH gene segments, one or more human D gene segments and one or more human JH gene segments into DNA of a chromosome 12 so that the human gene segments are operably connected upstream of a mouse or human endogenous heavy chain constant region (optionally Cmu and/or Cgamma);
(d) simultaneously or separately from step (c), deleting all or part of the mouse endogenous heavy chain VDJregion of said chromosome 12 to inactivate endogenous antibody heavy chain expression, wherein the deletion includes mouse ADAM 6-encoding nucleotide sequences;
(e) developing the EScell into a child mouse or progeny thereof whose genome comprises a said transgenic heavy chain locus; and
(f) by breeding using said child mouse (or progeny) and a further mouse whose genome comprisesone or more ADAM 6-encoding nucleotide sequences, developing a fertile mouse or a progeny thereof whose genome is homozygous for said transgenic heavy chain locus and encodes ADAM 6, wherein all or part of the endogenous heavy chain VDJregion has been deleted from both chromosomes 12 in the genome; optionally wherein said fertile mouse or progeny is male.

Disclosed herein is a fourth configuration, which is a fertile non-human vertebrate (optionally a male) that is homozygous for a transgenic antibody heavy chain locus, the vertebrate having a genome that
(i) comprises each transgenic heavy chain locus on a respective copy of a first chromosome; and
(ii) is inactivated for endogenous antibody heavy chain expression;
wherein each first chromosome of the genome comprises
(iii) a transgenic antibody heavy chain locuscomprising one or more human VH gene segments, one or more human D gene segments and one or more human JH gene segments operably connected upstream of a mouse or human heavy chain constant region (optionally Cmu and/or Cgam ma);
(iv) a deletion of all or part of the endogenous heavy chain VDJ region of said chromosome to inactivate endogenous antibody heavy chain expression, wherein the deletion includes ADAM 6; and
wherein the genome comprises
(v) an insertion of one or more expressible ADAM 6-encoding nucleotide sequences.

Thus, ADAM 6 resides on each said first chromosome in a wild-type fertile non-human vertebrate, but inactivation of endogenous heavy chain expression involves deletion of ADAM 6 that is co-located with the deleted heavy chain gene segments on the same chromosome. For example, use of homologous recombination precisely to replace endogenous heavy chain VDJwith human VDJgene segments as in the prior art deletes endogenous ADAM 6, thus affecting fertility. In the mouse, this happens when deletion of all or part of the endogenous heavy chain VDJregion on chromosome 12 is deleted to inactivate endogenous heavy chain expression. In the rat, this happens when deletion of all or part of the endogenous heavy chain VDJ region on chromosome 6 is deleted to inactivate endogenous heavy chain expression. Disclosed herein is insertion of ADAM 6 into the vertebrate genome in order to restore fertility.

In one aspect of the fourth configuration, the vertebrate is a mouse and each first chromosome is a chromosome 12.

Thus, in one aspect of the fourth configuration, the vertebrate isa rat and each first chromosome is a chromosome 6.

Disclosed herein is a method of making a fertile non-human vertebrate, eg, mouse or rat, that is homozygousfor a transgenic antibody heavy chain locus by carrying out steps (a) to (d) in an ES cell and using ES cell genome technology developing a final non-human vertebrate having a genome comprising an inserted ADAM 6-encoding nucleotide sequence (in homozygousor heterozygous state) and said transgenic heavy chain locus in homozygous state, wherein endogenous ADAM6 has been deleted. Disclosed herein is a fertile non-human vertebrate, eg, mouse or rat, that is made by this method, or a fertile male or female progeny thereof.

### BRIEF DESCRIPTION OF THE FIGURES

Figures 1 a & 1b: Schematic for endogenous IgH inactivation and retention of Adam6 by translocation;
Figure 2: Schematic for homologous recombination replacement of endogenous (mouse) IgH loci gene segments with human gene segments and accompanying deletion of Adam6 genes;
Figure 3: Schematic for RMGR replacement of endogenous (mouse) IgH loci gene segments with human gene segments and accompanying deletion of Adam6 genes;
Figure 4: Schematic for the creation and targeting of a deletion vector;
Figure 5: Schematic for the creation of a targeting vector containing Adam6 genes;
Figure 6: Schematic for the creation of IgH BACcontaining Adam6 genes.

### DETAILED DESCRIPTION OF THE INVENTION

Disclosed herein is a method of making a fertile non-human vertebrate (eg, a mouse) that is homozygous for a transgenic antibody heavy chain locus. The final mouse resulting from the method is in one aspect a male, so that the disclosure improves upon the prior art male transgenic mice that are infertile as a result of genomic manipulation. Fertile mice produce sperm that can fertilise eggs from a female mouse. Fertility is readily determined, for example, by successfully breeding to produce an embryo or child mouse. Disclosed herein is a method of making a final female mouse. Such females are, of course, useful for breeding to create male progeny carrying ADAM 6 and which are fertile.

In the method of this aspect of the disclosure, the final mouse has a genome that comprises each transgenic heavy chain locus on a respective copy of chromosome 12. The heavy chain loci in wild-type mice are found on chromosomes 12 and, as per the explanation below, disclosed herein is building a transgenic locus on the same chromosome. In one example, the transgenic locus is a chimaeric locus that comprises human VDJgene segments inserted upstream of the endogenous mouse constant region (at least the mouse Cmu and/or Cgamma). The human gene segments are operably connected with the constant regions in the present invention so that, after differentiation into a B-cell progeny in a mouse, the B-cell is able to express chimaeric antibodies comprising heavy chains having human variable regions and mouse constant regions. In an alternative aspect of any configuration of the disclosure, instead of a mouse (or non-human) constant region, each transgenic heavy chain locus comprises said human VDJgene segments operably connected upstream of a human heavy chain constant region, eg, human Cmu (optionally with a mouse or human Smu with human Cmu) and/or human gamma.

To this end, the method comprises the step of: constructing a transgenic mouse embryonic stem cell (ES cell) comprising a transgenic antibody heavy chain locus by inserting one or more human VH gene segments, one or more human D gene segments and one or more human JH gene segments into DNA of a chromosome 12 so that the human gene segments are operably connected upstream of a mouse endogenous heavy chain constant region (optionally Cmu and/or Cgamma). Optionally, the human gene segments are inserted upstream of the endogenous mouse Smu switch and Cmu. This is useful to harness the mouse endogenous regulatory control for class switching from IgM to another type (eg, IgG) antibodies *in vivo* following immunisation of a final mouse with an antigen of interest. In an example, the resultant ES cell is heterozygous for the transgenic heavy chain locus, ie, the transgenic locus is present on one chromosome 12 in the cell. The other chromosome 12 can, for example, bear the endogenous heavy chain locus and optionally this is inactivated (eg, by insertion of a functional marker (eg, neo or hprt) or by deletion of all or part of the locus, such as all or part of the endogenous VDJregion). The heterozygous EScell can be developed in due course into a mouse that is heterozygous for the heavy chain transgenic locus and using breeding and crossing with other mice also containing a copy of the transgenic heavy chain locus, a resultant progeny can be obtained that is homozygous for the transgenic heavy chain transgene. One or more ADAM 6-encoding nucleotide sequences can have been inserted (as described further below) into the genome of one or both of the heterozygous ancestor mice (eg, by insertion of ADAM6 into a respective ES cell that is an ancestor of the ancestor mouse; or by breeding of mice, one of which bearsADAM6, so that the resultant progeny is one of said ancestor mice bearing ADAM6). Alternatively, a progeny mouse that is homozygous for the heavy chain transgene but null for ADAM 6 can be crossed with a mouse whose genome contains an ADAM 6 gene, and using breeding a progeny that is homozygous for the heavy chain transgene and also contains an ADAM6 gene (in heterozygous or homozygous state) can be obtained. Instead of using just breeding, ES cell genome manipulation can be used to insert an ADAM 6-encoding nucleotide sequence into an ES cell derived from a progeny mouse that is homozygousfor the heavy chain transgene and a mouse subsequently is developed from the ES cell (or a progeny thereof) so that the final mouse genome is homozygous for the heavy chain transgene and also comprises an ADAM 6 gene. Techniques of animal husbandry, crossing, breeding, as well as EScell (eg, IPS cell) genome manipulation are readily available in the state of the art and will be familiar to the skilled person.

In the method of the present disclosure, simultaneously or separately from inserting the human gene segments into the ES cell genome, all or part of the mouse endogenous heavy chain VDJregion of said chromosome 12 is deleted to inactivate endogenous antibody heavy chain expression, ie, in a final progeny mouse derived from the ES cell, endogenous antibody heavy chain expression is inactivated. In one aspect, the endogenous VDJdeletion is carried out simultaneously with the insertion of the human VDJ. For example, one can use homologous recombination in a technique precisely to replace the entire mouse VDJregion (or part thereof including ADAM6-encoding nucleotide sequences) with the human VDJgene segments. One method (eg, see WO2002066630) is to use a plurality of homologous recombination vectors (eg, bacterial artificial chromosomes; BACs) each bearing one or more human VH and/or D and/or JH segments, in which a vector has homology armsflanking one or more human VH gene segmentsto be placed at the 5' end of the transgenic heavy chain locus. In this vector, the 5' homology arm can be a sequence corresponding to a mouse genomic sequence immediately 5' of the endogenous heavy chain locus. Using standard homologous recombination, this inserts the human gene segments precisely to replace endogenous mouse gene segments at the 5' position of the endogenous heavy chain locus. Another vector comprises homology arms flanking one or more human JH gene segments (and optionally all or part of the mouse J-C intron) to be placed at the 3' end of the transgenic heavy chain VDJ. In this vector, the 3' homology arm can be a sequence corresponding to a mouse genomic sequence immediately 5' of the endogenous heavy chain Cmu (or another downstream endogenous constant region); alternatively, the 3' homology arm can be a sequence corresponding to all or part of the endogenous J-C intron. Using standard homologous recombination, this inserts the human gene segments from this vector precisely to replace endogenous mouse gene segments at the 3' position of the endogenous heavy chain locus. In one aspect, the plurality of BACs have overlapping homology arms and can be used to replace the endogenous VDJwith human VDJgene segments, eg, see WO2009076464). In another aspect, one or more of these homologous recombination techniques can be generally used, with the modification that the human VDJis inserted immediately downstream (3') of the endogenous VDJ region (eg, inserted in the endogenous J-Cmu intron) and in one or more subsequent steps the endogenousVDJ(or part thereof comprising the ADAM 6-encoding nucleotide sequences) isdeleted, eg, using standard site-specific recombination (eg, cre/lox), transposon (eg, piggyBac transposon) or homologous recombination techniques. In another aspect, the human VDJis inserted 5' (eg, immediately 5' or within 100kb 5') of the first mouse VH gene segment and in one or more subsequent steps the endogenousVDJ(or part thereof comprising the ADAM 6-encoding nucleotide sequences) isdeleted.

In one aspect of any configuration of the method of the disclosure, the endogenous VDJ (or part thereof including ADAM 6-encoding nucleotide sequence(s)) isdeleted from the chromosome by translocation to a different chromosome species. For example, the different chromosome is chromosome 15. Translocation between chromosomes 12 and 15 in a mouse, for example, is desirable since it is known from published observations that translocation between the heavy chain locus on chromosome 12 and *c-mycon* chromosome 15 is possible (see, eg, Science 24 December 1982: Vol. 218 no. 4579 pp. 1319-1321; "Mouse c-myc oncogene is located on chromosome 15 and translocated to chromosome 12 in plasmacytomas"; Crews *et al*). Thus, in one example where the vertebrate is a mouse, the endogenous VDJ (or part thereof) is deleted from chromosome 12 by translocation to a chromosome 15. In another example, where the vertebrate is a rat, the endogenous VDJ(or part thereof) is deleted from chromosome 6 by translocation to a chromosome 15. Thus, in the final fertile mouse or mouse progeny disclosed herein, endogenous heavy chain expression is inactivated by translocation of at least part of the endogenous heavy chain loci VDJto a non-wild-type chromosome (ie, not a chromosome 12). Thus, in the final fertile rat or rat progeny disclosed herein, endogenous heavy chain expression is inactivated by translocation of at least part of the endogenous heavy chain loci VDJto a non-wild-type chromosome (ie, not a chromosome 6). In this case, the translocated endogenous VDJ (or part) is retained in the animal's genome, but is rendered non-functional for endogenous heavy chain expression. This is advantageous because the endogenous ADAM 6 genes are deleted from the wild-type chromosomal location to effect inactivation, but are then inserted into the genome elsewhere on an entirely different chromosomal species (ie, one not harbouring an antibody heavy chain locus) by translocation in a way that enables the inserted endogenous ADAM 6 genes to function (and thus give fertility in downstream animals) without re-activating endogenous heavy chain expression. Thus, translocation enables inactivation with concomitant retention of endogenous, wild-type ADAM 6 genes to provide for fertility in resultant animals. This perfectly tailors the ADAM 6 genes to the animal's genome (since it is the endogenous sequence), and also in one aspect enables transfer of each inserted endogenous ADAM 6 genes together with its endogenous promoter (and any other control elements such as enhancers). Thus, in one aspect inactivation is carried out by the deletion of a chromosomal sequence (eg, sequence of chromosome 12 in a mouse or 6 in a rat) comprising one or more ADAM 6 genes including respective promoter(s) and this is inserted by translocation to a chromosome that does not comprise a heavy chain locus (eg, in a mouse a chromosome other than a chromosome 12; in a rat achromosome other than a chromosome 6). This can be achieved, for example by translocating at least the DNA immediately flanked by the 3' most endogenous VH gene segment and the 5' most endogenous D segment. In one example, where the non-human vertebrate is a mouse, the translocated DNA comprises or constists of DNA from mouse V_{H}5-1 to D1-1 gene segments. In an aspect, the entire endogenous VD region is translocated; in another aspect the entire VDJregion is translocated, in either case this will also translocate the embedded endogenous ADAM6 genes.

All of the techniques described herein with reference to a mouse also apply to other non-human vertebrates where ADAM6 will be deleted along with endogenous VDJ, eg, where the ADAM6 is embedded in the endogenous VDJregion. For example, the techniques can be applied to another transgenic murine species. The techniquescan be applied to atransgenic rat. The disclosure, throughout, is to be read with this in mind, so that discussion relating to transgenic mice is equally applicable to making other non-human transgenic animals. Thus, for example, where a mouse chromosome 12 is mentioned and the making of a transgenic mouse, the disclosure herein can be read in the alternative to the making of atransgenic rat, and in thiscase rat chromosome 6 is intended.

In all cases, the deletion in the endogenous VDJ region on a chromosome preferably includes a deletion of all ADAM 6-encoding nucleotide sequences. Thus, when the vertebrate is a mouse, ADAM6a and ADAM6b are deleted. For example, the DNA immediately flanked by the 3' most endogenous VH gene segment and the 5' most endogenous D segment is deleted. In one example, where the non-human vertebrate is a mouse, the DNA from mouse V_{H}5-1 to D1-1 gene segments is deleted.

Disclosed herein is a method of making a fertile non-human vertebrate, eg, mouse or rat, that is homozygous for a transgenic antibody heavy chain locus by carrying out steps (a) to (d) in an EScell and using ES cell genome technology developing a final non-human vertebrate having a genome comprising an inserted ADAM 6-encoding nucleotide sequence (in homozygous or heterozygous state) and said transgenic heavy chain locus in homozygous state, wherein endogenous ADAM 6 has been deleted. Disclosed herein is a fertile non-human vertebrate, eg, mouse or rat, that ismade by this method, or a fertile male or female progeny thereof.

In one aspect, simultaneously or separately from inserting the human VDJand deleting the endogenous VDJ(or part thereof), the method comprises
- inserting into the ES cell genome one or more ADAM 6-encoding nucleotide sequences; and
- developing the ES cell into a fertile mouse or a progeny thereof whose genome is homozygous for said transgenic heavy chain locus and encodes ADAM 6, wherein all or part of the endogenous heavy chain VDJregion has been deleted from both chromosomes 12 in the genome; optionally wherein said fertile mouse or progeny is male.

In another aspect, after inserting the human VDJand deleting the endogenous VDJ (or part thereof), the method comprises
- developing the ES cell into a child mouse or progeny thereof whose genome comprises one or more of said transgenic heavy chain locus (eg, is homozygous for the transgenic heavy chain locus);
- deriving a second ES cell from said mouse and inserting into the genome of said second ES cell one or more ADAM 6-endcoding nucleotide sequences; and
- developing the second EScell into a fertile mouse or a progeny thereof whose genome is homozygous for said transgenic heavy chain locus and encodes ADAM 6, wherein all or part of the endogenous heavy chain VDJ region has been deleted from both chromosomes 12 in the genome; optionally wherein said fertile mouse or progeny is male.

In another aspect, after inserting the human VDJand deleting the endogenous VDJ(or part thereof), the method comprises
- developing the ES cell into a child mouse or progeny thereof whose genome comprises a said transgenic heavy chain locus (eg, is homozygous for the transgenic heavy chain locus); and
- by breeding using said child mouse (or progeny) and a further mouse whose genome comprises one or more ADAM 6-encoding nucleotide sequences, developing a fertile mouse or a progeny thereof whose genome is homozygous for said transgenic heavy chain locus and encodes ADAM 6, wherein all or part of the endogenous heavy chain VDJ region has been deleted from both chromosomes 12 in the genome; optionally wherein said fertile mouse or progeny is male.

In this aspect, optionally said further mouse is homozygous for ADAM 6, eg, the mouse genome comprises ADAM 6a and ADAM6b in homozygous state. Optionally said mice are of the same mouse strain.

The skilled person will be aware of techniques for deriving embryonic stem cells. For example, said second EScell can be generated from an embryo (eg, blastocyst stage) using any standard technique for EScell generation. For example, reference is made to Proc Natl Acad Sci 1997 May 27; 94(11):5709-12; "The origin and efficient derivation of embryonic stem cells in the mouse"; Brook FA & Gardner RL. The embryo can be said child mouse or a progeny embryo thereof. Other standard ES cell-generating techniques can be used. In another aspect, the second ES cell is an IPScell (induced pluripotent stem cell) that is derived from said child mouse or progeny thereof. Reference is made to WO2007069666, WO2008118820, WO2008124133, WO2008151058, WO2009006997 and WO2011027180, which provide guidance on IPStechnology and suitable methods. The IPS cell can in one example be directly generated (ie, without need for breeding) from a somatic cell of the child mouse or a progeny mouse thereof using standard methods.

The skilled person conversant with ES cell technology will readily know how to develop a child from a transgenic EScell whose genome has been manipulated. For example, a non-human (eg, mouse) ES cell (such as an EScell comprising a heavy chain transgenic locus) is implanted into a donor blastocyst (eg, a blastocyst of the same strain of vertebrate as the EScell). The blastocyst is then implanted into a foster mother where it develops into a child (embryo or a born child). In this way, a plurality of children can be developed, each from a respective modified child ES cell. Siblings can be bred together to achieve crosses providing one or more resultant progeny that are homozygous for the transgenic heavy chain locus.

In one example, a mouse ES cell according to any configuration, aspect or example of the disclosure is developed into a child or progeny by
(f) transferring the EScell into a donor mouse blastocyst or earlier-stage embryo (eg, pre-morula stage);
(g) implanting the blastocyst or embryo into a foster mouse mother; and
(h) developing the blastocyst or embryo into a child mouse or progeny thereof that is fertile and whose genome is homozygous for said transgenic heavy chain locus and encodes ADAM 6.

In any aspect of a configuration for use in the method of the invention, the position of insertion of ADAM 6-encoding nucleotide sequence(s) is not limited to the original chromosome (eg, chromosome 12 for a mouse or chromosome 6 for a rat); insertion into another chromosome is possible, or on the original chromosome but spaced away from the wild-type ADAM 6 gene location. In an example, an ADAM6 gene is inserted into an original chromosome, eg, when making a transgenic mouse, an ADAM 6-encoding nucleotide sequence is inserted into a chromosome 12; when making a transgenic rat, an ADAM 6-encoding nucleotide sequence is inserted into a chromosome 6. In one example, an ADAM 6-encoding nucleotide sequence is inserted within 20, 15, 10, 5, 4, 3, 2, 1 or 0.5 Mb of one or both transgenic heavy chain loci. This is useful to maximise linkage between the inserted ADAM 6 and the transgenic heavy chain locus, to minimise separation of the genes during subsequent meiosis and crossing, eg, during breeding of progeny. Thus, final mice and progeny thereof can retain the fertility advantage of the invention while permitting useful subsequent breeding and crossing to create new animal lines. In another example, an ADAM 6-encoding nucleotide sequence is inserted within one or both transgenic heavy chain loci, eg, in the DNA between the 3' most human VH gene segment and the 5' most human D segment, which nature indicates as a permissive permission for harbouring ADAM6.

In any aspect of a configuration for use in the method of the invention, one or more ADAM 6 (eg, two)-encoding nucleotide sequences are inserted into the vertebrate genome by EScell technology and/or by breeding. The inserted ADAM 6-encoding nucleotide sequence(s) do not need to be from the same species as the recipient non-human vertebrate. For example, the vertebrate is a mouse and a rat or primate (eg, human) ADAM 6-encoding nucleotide sequence is inserted. For example, the vertebrate is a rat and a mouse or primate (eg, human) ADAM 6-encoding nucleotide sequence is inserted.

In one aspect, the vertebrate is a mouse and an ADAM 6-encoding nucleotide sequence is inserted on one or both chromosomes 12. For example, mouse ADAM 6a and ADAM 6b or rat ADAM 6 is inserted on one or both chromosomes 12. For example, a mouse ADAM 6-encoding nucleotide sequence is inserted between the 3' most human VH gene segment and the 5' most human D segment.

In one aspect, the vertebrate is a rat and an ADAM 6-encoding nucleotide sequence is inserted on one or both chromosomes 6. For example, mouse or rat ADAM 6 is inserted on one or both chromosomes 6. For example, a mouse or rat ADAM 6-encoding nucleotide sequence is inserted between the 3' most human VH gene segment and the 5' most human D segment.

In any aspect of a configuration for use in the method of the invention, each ADAM6 is expressible. For example, the inserted ADAM 6 nucleotide sequence is inserted so that it is operably connected to a promoter (and optionally an enhancer or other regulatory element) for expression. The promoter can be one that is endogenous to the non-human vertebrate, eg, a mouse promoter (eg, one that drives ADAM 6 expression in wild-type mice), or it can be exogenous (from a different species). For example, the inserted ADAM 6 in the genome is a rat ADAM 6 nucleotide sequence operably connected to an endogenous mouse ADAM 6 promoter. Alternatively, the inserted ADAM6 in the genome is a mouse ADAM 6 nucleotide sequence operably connected to an endogenous rat ADAM 6 promoter.

In one aspect, an ADAM6 nucleotide sequence is inserted which is selected from the group consisting of SEQ ID NO: 1, 2, 3 and 4 (see sequence listing below).

In one aspect of the method of the invention, the human immunoglobulin gene segments are inserted into the chromosome to replace all or part of the endogenous heavy chain VDJregion, so that insertion of the human gene segments and deletion of the endogenous VDJDNA from the chromosome or genome take place simultaneously; optionally wherein the entire endogenous VDJ region is replaced. Insertion of the human gene segments is, for example, performed using homologous recombination and/or site-specific recombination (eg, recombinase mediated cassette exchange) to execute the precise replacement. Deletion of the endogenous VDJ(and particularly the entire endogenous VDJ) from the genome is advantageous to totally eliminate the possibility of recombination with constant region gene segments, thus totally eliminating endogenous heavy chain expression with certainty.

In one example of the method of the invention, wherein the vertebrate is a mouse, mouse ADAM6a and ADAM 6b-encoding nucleotide sequences are inserted, such that the final fertile mouse can express both ADAM 6a and ADAM 6b proteins.

In one example of the method of the invention, the genome of the final fertile mouse or progeny is homozygous for each inserted ADAM 6-encoding nucleotide sequence. Optionally the genome comprises more than two copies of mouse ADAM6a and/or ADAM 6b-encoding nucleotide sequences. Optionally, as an alternative, the genome comprises 2 copies of ADAM 6a and one copy (heterozygous) of ADAM 6b; or one copy of ADAM 6a and 2 copies of ADAM 6b.

In another configuration, the disclosure provides a fertile non-human vertebrate (optionally a male) that is homozygous for a transgenic antibody heavy chain locus, the vertebrate having a genome that
(i) comprises each transgenic heavy chain locus on a respective copy of a first chromosome; and
(ii) is inactivated for endogenous antibody heavy chain expression;
   wherein each first chromosome of the genome comprises
(iii) a transgenic antibody heavy chain locus comprising one or more human VH gene segments, one or more human D gene segments and one or more human JH gene segments operably connected upstream of a mouse heavy chain constant region (optionally Cmu and/or Cgamma);
(iv) a deletion of all or part of the endogenous heavy chain VDJregion of said chromosome to inactivate endogenous antibody heavy chain expression, wherein the deletion includes ADAM 6; and
   wherein the genome comprises
(v) an insertion of one or more expressible ADAM 6-encoding nucleotide sequences.

For example, the non-human vertebrate is murine. For example, the non-human vertebrate is a mouse or a rat.

In one aspect, the disclosure provides a non-human vertebrate such as a mouse (optionally a male mouse) that is homozygous for a transgenic antibody heavy chain locus, the mouse having a genome that
(i) comprises each transgenic heavy chain locus on a respective copy of chromosome 12 (or equivalent chromosome for said vertebrate); and
(ii) is inactivated for endogenous antibody heavy chain expression;
   wherein each chromosome 12 of the genome comprises
(iii) a transgenic antibody heavy chain locuscomprising one or more human VH gene segments, one or more human D gene segments and one or more human JH gene segments operably connected upstream of a heavy chain constant region (optionally Cmu and/or Cgamma);
(iv) a deletion of all or part of the mouse endogenous heavy chain VDJ region of said chromosome 12 to inactivate endogenousantibody heavy chain expression (later when differentiated into a mouse/B cells), wherein the deletion includes mouse ADAM 6-encoding nucleotide sequences (ie, no functional endogenous ADAM 6 genes remain in the genome); and
   wherein the genome comprises
(v) an insertion of one or more expressible ADAM 6-encoding nucleotide sequences.

The considerations of how and where to insert ADAM 6 sequences in the animals disclosed herein are addressed generally above.

The constant region is, eg, a mouse constant region, eg, an endogenous constant region. Thus, when the vertebrate is a mouse, the constant region is an endogenous mouse constant region, eg, a mouse Cmu and/or a mouse Cgamma, optionally with an endogenous mouse or rat Smu switch.

In another aspect, the disclosure provides a non-human rat (optionally a male rat) that is homozygous for a transgenic antibody heavy chain locus, the rat having a genome that
(i) comprises each transgenic heavy chain locus on a respective copy of chromosome 6; and
(ii) is inactivated for endogenous antibody heavy chain expression;
   wherein each chromosome 6 of the genome comprises
(iii) a transgenic antibody heavy chain locus comprising one or more human VH gene segments, one or more human D gene segments and one or more human JH gene segments operably connected upstream of a heavy chain constant region (optionally Cmu and/or Cgamma);
(iv) a deletion of all or part of the rat endogenous heavy chain VDJregion of said chromosome 6 to inactivate endogenous antibody heavy chain expression (later when differentiated into a mouse/B cells), wherein the deletion includes rat ADAM 6-encoding nucleotide sequences (ie, no functional endogenous ADAM 6 genes remain in the genome); and
   wherein the genome comprises
(v) an insertion of one or more expressible ADAM 6-encoding nucleotide sequences.

The constant region is, eg, a rat constant region, eg, an endogenous constant region. Thus, when the vertebrate is a rat, the constant region is an endogenous rat constant region, eg, a rat Cmu and/or a rat Cgamma, optionally with an endogenous mouse or rat Smu switch.

In one example of the homozygous mouse or rat of the disclosure, each inserted ADAM 6-encoding nucleotide sequence is on a (i) chromosome 12 wherein the animal is a mouse; or (ii) chromosome 6 wherein the animal is a rat.

In one example of the homozygous mouse or rat of the disclosure, an inserted ADAM 6-encoding nucleotide sequence is inserted (i) within one or both transgenic heavy chain loci or (ii) within 20 Mb of one or both transgenic heavy chain loci.

In one example of the homozygous mouse or rat of the disclosure, the human gene segments replace all or part of the endogenous VDJ region in each heavy chain locus.

In one example of the homozygous mouse or rat of the disclosure, the genome comprises inserted expressible mouse ADAM 6a and ADAM 6b-encoding nucleotide sequences.

In one example of the homozygous mouse or rat of the disclosure, the genome comprises an inserted expressible rat ADAM 6-encoding nucleotide sequence.

In one example of the homozygous mouse or rat of the disclosure, the genome is homozygous for each inserted ADAM 6-encoding nucleotide sequence. Optionally the genome comprises more than two copies of ADAM 6-encoding nucleotide sequences selected from rat ADAM6, mouse ADAM6a and mouse ADAM 6b-encoding nucleotide sequences. Optionally, the genome comprises 2 copies of ADAM 6a and one copy (heterozygous) of ADAM 6b; or one copy of ADAM 6a and 2 copies of ADAM 6b.

In one example of the homozygous mouse or rat of the disclosure, the genome comprises one or more transgenic light chain loci each comprising one or more human light chain V gene segments and one or more light chain Jgene segments operably connected upstream of a light chain constant region (eg, an endogenous mouse or rat C kappa constant region).

### Inactivation of Endogenous Antibody Chain Expression by Translocation

In one configuration, disclosed herein is:-

A non-human vertebrate (optionally a mouse or rat) or non-human vertebrate cell (optionally a mouse or rat cell) having a genome that
(i) comprises one or more transgenic antibody loci capable of expressing antibodies comprising human variable regions (optionally following antibody gene rearrangement); and
(ii) is inactivated for endogenous antibody expression;
   wherein
(iii) endogenous variable region gene segments have been translocated to a chromosomal species (eg, chromosome 15) that does not contain antibody variable region gene segments in wild-type vertebrates of said non-human type, whereby endogenous antibody expression is inactivated.

The vertebrate can be any non-human vertebrate species disclosed herein. The transgenic antibody loci can be according to any one disclosed herein. The cell can be an ES cell, IPS cell, Cell or any other non-human vertebrate cell disclosed herein.

In an example, the endogenous variable region gene segments have been translocated to a chromosomal species (eg, chromosome 15) that does not contain antibody variable region gene segments in wild-type vertebrates of said non-human type by translocation in an ancestor cell (eg, an EScell) from which the vertebrate or cell disclosed herein is derived.

Disclosed herein is:-
A mouse or mouse cell having a genome that
   (i) comprises one or more transgenic antibody loci capable of expressing antibodies comprising human variable regions (optionally following antibody gene rearrangement); and
   (ii) is inactivated for endogenous mouse antibody expression;
      wherein
   (iii) a plurality of endogenous mouse variable region gene segments are absent from chromosomes 12 in the genome, but are present in germline configuration (with respect to each other) on one or more chromosomesother than chromosomes 12 (eg, the gene segments are on chromosome 15), whereby endogenous mouse antibody expression is inactivated.

Furthermore, disclosed herein is:-
A rat or rat cell having a genome that
(i) comprisesone or more transgenic antibody loci capable of expressing antibodies comprising human variable regions (optionally following antibody gene rearrangement); and
(ii) is inactivated for endogenous rat antibody expression;
   wherein
(iii) a plurality of endogenous rat variable region gene segments are absent from chromosomes 6 in the genome, but are present in germline configuration (with respect to each other) on one or more chromosomes other than chromosomes 6 (eg, the gene segments are on chromosome 15), whereby endogenous rat antibody expression is inactivated.

When the disclosure relates to a cell, such as an ES cell, inactivation of endogenous antibody expression relates to the inability of a differentiated antibody-producing progeny cell or non-human vertebrate to express endogenous antibodies, ie, antibodies whose variable regions are only of said non-human vertebrate type (eg, mouse or rat antibodies) and not human variable regions. Thus, in the present disclosure, the vertebrate, mouse, rat only expresses transgenic antibodies that comprise human variable regions and does not (or not substantially) express endogenous antibodies. The transgenic antibody loci may, in an example, undergo rearrangement *in vivo,* eg, following immunisation of the vertebrate, mouse or rat with a predetermined antigen. Following rearrangement, the organism is capable of expressing antibody chains from said rearranged loci, which chains form antibodies comprising human variable regions.

In an aspect, the vertebrate, mouse, rat or cell genome comprises a transgenic antibody heavy chain locus (in heterozygous or homozygous state), the locus comprising one or more human VH gene segments, one or more human D gene segments and one or more human JH gene segments operably connected upstream of a non-human vertebrate (eg, mouse or rat) constant region (optionally Cmu and/or Cgamma); and said endogenous variable region gene segments are selected from endogenous
(a) VH;
(b) D;
(c) JH;
(d) VH and D;
(e) D and JH; and
(f) VH, D and JH.

In (a) to (f), intervening sequences between gene segmentscan be included.

In an example of this aspect, the constant region is an endogenous constant region, eg, endogenous Cmu and/or Cgamma, such as endogenous mouse Cmu and/or endogenous mouse Cgamma.

In an aspect, the vertebrate, mouse, rat or cell genome comprises expressible endogenous ADAM6 gene(s) or ADAM 6-encoding nucleotide sequence(s).

In an aspect, the vertebrate, mouse, rat or cell is a male vertebrate, mouse, rat or cell, eg, one whose genome comprises endogenous ADAM 6 gene(s).

In one aspect, substantially the entire endogenous VDJ(or part thereof including ADAM 6-encoding nucleotide sequence(s)) is deleted from the chromosome by translocation to a different chromosome species. For example, the different chromosome ischromosome 15. Translocation between chromosomes 12 and 15 in a mouse, for example, is desirable since it is known from published observations that translocation between the heavy chain locus on chromosome 12 and c-mycon chromosome 15 ispossible (see, eg, Science 24 December 1982: Vol. 218 no. 4579 pp. 1319-1321; "Mouse c-myc oncogene is located on chromosome 15 and translocated to chromosome 12 in plasmacytomas"; Crews *et al*). Thus, in one example where the vertebrate is a mouse, the endogenous VDJ(or part thereof) isdeleted from chromosome 12 by translocation to a chromosome 15. In another example, where the vertebrate is a rat, the endogenous VDJ (or part thereof) is deleted from chromosome 6 by translocation to a chromosome 15. Thus, in the final fertile mouse or progeny disclosed herein, endogenous heavy chain expression is inactivated by translocation of at least part of the endogenous heavy chain loci VDJto a non-wild-type chromosome (ie, not a chromosome 12). Thus, in the final fertile rat or progeny disclosed herein, endogenous heavy chain expression is inactivated by translocation of at least part of the endogenous heavy chain loci VDJto a non-wild-type chromosome (ie, not achromosome 6). In this case, the translocated endogenous VDJ (or part) is retained in the animal's genome, but is rendered non-functional for endogenous heavy chain expression. This is advantageous because the endogenous ADAM 6 genes are deleted from the wild-type chromosomal location to effect inactivation, but are then inserted into the genome elsewhere on an entirely different chromosomal species (ie, one not harbouring an antibody heavy chain locus) by translocation in a way that enables the inserted endogenous ADAM 6 genes to function (and thus give fertility in downstream animals) without re-activating endogenous heavy chain expression. Thus, translocation enables inactivation with concomitant retention of endogenous, wild-type ADAM 6 genes to provide for fertility in resultant animals. This perfectly tailors the ADAM 6 genes to the animal's genome (since it is the endogenous sequence), and also in one aspect enables transfer of each inserted endogenous ADAM6 genes together with its endogenous promoter (and any other control elements such as enhancers). Thus, in one aspect inactivation is carried out by the deletion of a chromosomal sequence (eg, sequence of chromosome 12 in a mouse or 6 in a rat) comprising one or more ADAM6 genes including respective promoter(s) and this is inserted by translocation to a chromosome that does not comprise a heavy chain locus (eg, in a mouse a chromosome other than a chromosome 12; in a rat a chromosome other than achromosome 6). This can be achieved, for example by translocating at least the DNA immediately flanked by the 3' most endogenous VH gene segment and the 5' most endogenous D segment. In one example, where the non-human vertebrate is a mouse, the translocated DNA comprises or consists of DNA from mouse V_{H}5-1 to D1-1 gene segments. In an aspects, the entire endogenous VD region is translocated; in another aspect the entire VDJ region is translocated, in either case this will also translocate the embedded endogenous ADAM 6 genes.

Thus, the disclosure provides:-
A method of making a non-human vertebrate cell (optionally a mouse or rat cell) or a non-human vertebrate (eg, a mouse or rat), the method comprising
   (i) inserting into a non-human ES cell genome one or more transgenic antibody loci comprising human variable region gene segments; and
   (ii) inactivating endogenous antibody expression by translocating endogenous variable region gene segments (eg, an entire endogenous heavy chain VDJ region) to a chromosomal species (eg, chromosome 15) that does not contain antibody variable region gene segments in wild-type vertebrates of said non-human type;
      whereby a non-human vertebrate EScell is produced that is capable of giving rise to a progeny cell (eg, a B-cell or hybridoma) in which endogenous antibody expression is inactivated and wherein the progeny is capable of expressing antibodies comprising human variable regions; and
   (iii) Optionally differentiating said ES cell into said progeny cell or a non-human vertebrate (eg, mouse or rat) comprising said progeny cell.

In an example, an entire (or substantially entire) endogenous heavy chain VDJregion including intervening sequences in germline configuration is translocated. Optionally, the genome of the cell/ vertebrate is homozygous for this translocation. Alternatively or additionally, a light chain VJ region is translocated, eg, an entire (or substantially entire) endogenous light chain (eg, kappa) VJ region including intervening sequences in germline configuration is translocated.

Non-human vertebrates disclosed herein are useful for generating antibodiesfollowing immunisation with a target antigen or epitope of interest. Usefully, the antibodies that are generated have human heavy chain (and optionally also light chain) variable regions. The heavy chain (and optionally light chain) constant regions are of the non-human species, eg, endogenous to the animal, this allows for harnessing of the endogenous antibody expression and B-cell development control mechanisms, thereby enhancing antibody generation. After isolation following antigen immunisation, a selected antibody can be formatted by swapping the constant region for a human constant region by conventional techniques to increase compatibility for human administration.

The antibodies isolated from the animals disclosed herein (or derivative antibodies) be of any format provided that they comprise human heavy chain variable regions. For example, the present invention is applicable to of 4-chain antibodies, where the antibodies each contain 2 heavy chains and 2 light chains. Alternatively, the invention can be applied to H2 antibodies (heavy chain antibodies) bearing human V regions and which are devoid of CH1 and light chains (equivalent in respectsto Camelid H2 antibodies: see, eg, Nature. 1993 Jun 3;363(6428):446-8; Naturally occurring antibodies devoid of light chains; Hamers-Casterman C, Atarhouch T, Muyldermans S, Robinson G, Hamers C, Songa EB, Bendahman N, Hamers R). These antibodies function to specifically bind antigen, such antibodies being akin to those found in the blood of *Camelidae* (eg, llamas, camels, alpacas). Such antibodies with human VH pairs can be synthetically produced to provide therapeutic and prophylactic medicaments (eg, see WO1994004678, WO2004041862, WO2004041863). Transgenic mice also can produce such heavy chain antibodies and the *in vivo* production of the antibodies allows the mouse's immune system to select for human VH-VH pairings, sometimes selecting for such pairings in which mutations have been introduced *in vivo* by the mouse to accommodate the pairing (WO2010109165A2). Thus, in an aspect of the present disclosure, the heavy chain transgene is devoid of a CH1 gene segment and the genome comprises no functional antibody light chain locus. Alternatively, the test antibody is an antibody fragment, eg, Fab or Fab₂, which comprises a constant region and human heavy chain variable regions.

The skilled person will be familiar with routine methods and protocols for immunising with antigen, eg, using prime and boost immunisation protocols. A suitable protocol isRIMMS(see Hybridoma 1997 Aug;16(4):381-9; "Rapid development of affinity matured monoclonal antibodies using RIM MS'; Kilpatrick *et al*). For immunisation of a vertebrate disclosed herein a suitable human target or epitope can be from any suitable source, eg, obtained by cloning the DNA from a blood or tissue sample of a human donor.

Throughout this text, and with application to any configuration, aspect, embodiment or example of the invention, the term "endogenous" (eg, endogenous constant region) in relation to a non-human vertebrate or cell, element or feature thereof (eg, "endogenous ADAM 6" or "endogenous constant region") indicates that the element is a type of element that is normally found in the vertebrate or cell of that non-human species or strain (as opposed to an exogenous constant region, ADAM6 or other element whose sequence is not normally found in such a vertebrate or cell).

In one example, each mouse or ES cell is one having a 129 mouse genetic background. In one example, the mouse or EScell has an AB2.1 mouse genetic background. In another example, the mouse or ES cell has a genetic background of a mouse strain selected from 129, C5 7BL/6N, C57BL/6J, JM 8, AB2.1, AB2.2, 129S5 or 129Sv.

An antibody isolated from a vertebrate disclosed herein can be subsequently derivatised, eg, by the addition (such as by chemical conjugation) of a label or toxin, PEG or other moiety, to make a pharmaceutical product. Derivatisation is useful, for example, when it isdesirable to add an additional functionality to the drug to be developed from the antibody. For example, for cancer indications it may be desirable to add additional moieties that assist in cell-killing. In another aspect, the variable regions of the antibody isolated from the vertebrate are affinity matured *in vivo* or *in vitro* (eg, by phage display, ribosome display, yeast display, *etc*). In another aspect, the constant regions of the antibody isolated from the vertebrate are mutated *in vivo* or *in vitro* (eg, by random or directed, specific mutation and optional selection by phage display, ribosome display, yeast display, *etc*). The constant region may be mutated to ablate or enhance Fc function (eg, ADCC).

In one aspect, the genome of the final vertebrate comprises one or more light chain antibody loci comprising human VJgene segments, eg, as described in any of WO2011004192, US7501552, US6673986, US6130364, WO2009076464 and US6586251. In one example, the final vertebrate comprises
(a) Heavy chain loci, each comprising one or more human heavy chain V gene segments, one or more human heavy chain D gene segments and one or more human heavy chain JH gene segments upstream of an endogenous non-human vertebrate (eg, endogenous mouse or rat) constant region (eg, Cmu and/or Cgamma);
(b) A kappa light chain locus (optionally in homozygous state) comprising one or more human kappa chain V gene segments, and one or more human kappa chain Jk gene segments upstream of an endogenous non-human vertebrate (eg, endogenous mouse or rat) kappa constant region; and optionally
(c) A lambda light chain locus (optionally in homozygous state) comprising one or more human lambda chain V gene segments, and one or more human lambda chain Jλ gene segments upstream of a lambda constant region; and
(d) Wherein the vertebrate is capable of producing chimaeric antibodies following rearrangement of said loci and immunisation with an antigen.

As is conventional in the art, there are provided methods for generating IPScells. For example, mouse embryo fibroblasts can be generated from a mouse embryo and then IPS cells generated using any standard technique. For example, reference is made to Proc Natl Acad Sci; 2011 Oct 11; "Rapid and efficient reprogramming of somatic cells to induced pluripotent stem cells by retinoic acid receptor gamma and liver receptor homolog 1"; Wang *et al*. Other standard IPS-generating techniques can be used.

In one aspect of any aspect of the disclosure, when an IPScells is used, the IPS cell is a mouse embryonic fibroblast cell.

Human DNA (eg, as a source of heavy and/or light chain gene segments) is readily obtainable from commercial and academic libraries, eg, Bacterial Artificial Chromosome (BAC) libraries containing human DNA. Examples are the Human RPCI-11 and -13 libraries (Osoegawa *et al*, 2001 -see below; http://bacpac.med.buffalo.edu/11framehmale.htm) and also the "CalTech" Human BAC libraries (CalTech Libraries A, B, Cand/or D, http://www.tree.caltech.edullib status.html).

### CalTech human BAC library D:

### See: http://www.ncbi.nlm.nih.gov/clone/library/genomic/16/

The Hiroaki Shizuya laboratory at the California Institute of Technology has developed three distinct human BAC libraries (obtainable from Open Biosystems). The Cal Tech B (CTB) and Cal Tech C(CTC) libraries together represent a genomic coverage of 15X. The Cal Tech D (CTD) library represents a 17X coverage of the human genome. Whole collections as well as individual clones are available. Detailed information on the construction of the libraries can be found at http://informa.bio.caltech.edu/idx www tree.html.

### Library Summary

Library Name: CalTech human BAC library D
Library Abbreviation: CTD
Organism: Homo sapiens
Distributors: Invitrogen, Open Biosystems
Vector type(s): BAC
# clones Clone DB: 226,848
# end sequences Clone DB: 403,688
# insert sequences Clone DB: 3,153
# clones with both ends sequenced: 153,035

### Library Details

| **DNA Source:** | **Sex** | **Cell type** | |
|---|---|---|---|
| | male | Sperm | |
| **Library Construction** | **Library segment** | **Vector Name** | **Vector Cloning Site(s)** |
| | 1 | pBeloBACII | Hindlll |
| | 2-5 | pBeloBACII | EcoRI |
| **Library Statistics** | **Library segment** | **Avg InSert (kb)** | **Plate Range(s)** |
| | 1 | 129 | 2001 to 2423 |
| | 2 | 202 | 2501 to 2565 |
| | 3 | 182 | 2566 to 2671 |
| | 4 | 142 | 3000 to 3253 |
| | 5 | 166 | 3254 to 4869 |

### RPCI-11 BACs

### References

- Osoegawa K, M ammoser AG, Wu C, Frengen E, Zeng C, Catanese JJ, de Jong PJ; Genome Res. 2001 Mar;1(3):483-96; "A bacterial artificial chromosome library for sequencing the complete human genome";
- Osoegawa, K, Woon, P.Y., Zhao, B., Frengen, E., Tateno, M., Catanese, J.J, and de Jong, P.J. (1998); "An Improved Approach for Construction of Bacterial Artificial Chromosome Libraries"; Genomics 52, 1-8;
- http://bacpac.chori.org/ hmale11.htm, which describes the BACs as follows

### BAC Availability

The RP11 BACs are available for purchase from Invitrogen (see
http://tools.invitrogen.com/content/sfs/manuals/bac clones man.pdf).

Vectors, such as BACs or PACs, can be manipulated *in vitro* by standard Molecular Biology techniques, for example recombineering (see http://www.genebridges.com; EP129142 and EP1204740). For example, recombineering can be used to create vectors in which a nucleotide sequence coding for human DNA of interest is flanked by one or more sequences, such as homology arms or site-specific recombination sites (eg, lox, frt or rox). The homology arms are, in one aspect, homologous to, or identical to, stretches of DNA from the genome of the non-human vertebrate to be used to generate the vertebrate. Vectors created in thisway are useful for performing homologous recombination (see, eg, US6638768) in a method of precisely inserting the human DNA into the non-human vertebrate genome (eg, to precisely replace the orthologous or homologous DNA in the vertebrate genome).

Other useful DNA- and genome-manipulation techniques are readily available to the skilled person, including technologies described in US6461818 (Baylor College of Medicine), U96586251 (Regeneron) and WO2011044050 (eg, see Examples).

Techniques for constructing non-human vertebrates and vertebrate cells whose genomes comprise a transgene, eg, a transgenic antibody locus containing human V, J and optionally D regions are well known in the art. For example, reference is made to WO2011004192, US7501552, US6673986, US6130364, WO2009/076464 and US6586251.

All nucleotide coordinates for the mouse are from NCBI m37, April 2007 ENSEMBL Release 55.37h for the mouse C57BL/6J strain. Human nucleotides are from GRCh37, Feb 2009 ENSEM BL Release 55.37 and rat from RGSC 3.4 Dec 2004 ENSEM BL release 55.34w.

In one aspect of a vertebrate disclosed herein, the vertebrate is a mammal, eg, a rodent. In one aspect of a vertebrate of the disclosure, the vertebrate is a mouse, rat, rabbit, Camelid (eg, a llama, alpaca or camel) or shark.

In one aspect the transgenic antibody loci comprise human V, D and/or Jcoding regions placed under control of the host regulatory sequences or other (non-human, non-host) sequences. In one aspect reference to human V, D and/or Jcoding regions includes both human introns and exons, or in another aspect simply exons and no introns, which may be in the form of cDNA.

Alternatively it is possible to use recombineering, or other recombinant DNA technologies, to insert a non human-vertebrate (e.g. mouse) promoter or other control region, such as a promoter for a V region, into a BACcontaining a human Ig region. The recombineering step then places a portion of human DNA under control of the mouse promoter or other control region.

Disclosed herein is a cell line (eg, ESor IPS cell line) which is grown from or otherwise derived from cells or a vertebrate asdescribed herein, including an immortalised cell line. The cell line may be immortalised by fusion to a tumour cell to provide an antibody producing cell and cell line, or be made by direct cellular immortalisation.

In one aspect the non-human vertebrate of any configuration of the disclosure is able to generate a diversity of at least 1 X 10⁶ different functional chim aeric antibody sequence combinations.

Optionally in any configuration for use in the method of the invention the constant region is endogenous to the vertebrate and optionally comprises an endogenous switch. In one aspect, the constant region comprises a Cgamma (Cγ) region and/or a Smu (Sµ) switch: Switch sequences are known in the art, for example, see Nikaido et al, Nature 292: 845-848 (1981) and also WO2011004192, US7501552, US6673986, US6130364, WO2009/076464 a US6586251, eg, SEQ ID NOs: 9-24 disclosed in US7501552. Optionally the constant region comprises an endogenous S gamma switch and/or an endogenous Smu switch.

In one optional aspect where the Vertebrate is a mouse, the insertion of the human antibody gene DNA, such as the human VDJ region is targeted to the region between the J4 exon and the Cµ locus in the mouse genome IgH locus, and in one aspect is inserted between coordinates 114,667,090 and 114,665,190, suitably at coordinate 114,667,091. In one aspect the insertion of human light chain kappa VJ is targeted into mouse chromosome 6 between coordinates 70,673,899 and 70,67.5,515, suitably at position 70,674,734, or an equivalent position in the lambda mouse locus on chromosome 16.

Reference to location of the variable region upstream of the non-human vertebrate constant region means that there is a suitable relative location of the two antibody portions, variable and constant, to allow the variable and constant regions to form a chimaeric antibody or antibody chain *in vivo* in the vertebrate. Thus, the inserted human antibody DNA and host constant region are in operable connection with one another for antibody or antibody chain production.

In one aspect the inserted human antibody DNA is capable of being expressed with different host constant regions through isotype switching. In one aspect isotype switching does not require or involve trans switching. Insertion of the human variable region DNA on the same chromosome as the relevant host constant region means that there is no need for trans-switching to produce isotype switching.

In the present disclosure, optionally at least one non-human vertebrate enhancer or other control sequence, such as a switch region, is maintained in functional arrangement with the non-human vertebrate constant region, such that the effect of the enhancer or other control sequence, as seen in the host vertebrate, is exerted in whole or in part in the transgenic animal. This approach is designed to allow the full diversity of the human locus to be sampled, to allow the same high expression levels that would be achieved by non-human vertebrate control sequences such as enhancers, and is such that signalling in the B-cell, for example isotype switching using switch recombination sites, would still use non-human vertebrate sequences.

A non-human vertebrate having such a genome would produce chimaeric antibodies with human variable and non-human vertebrate constant regions, but these are readily humanized, for example in a cloning step that replaces the mouse constant regions for corresponding human constant regions.

In one aspect the inserted human IgH VDJregion comprises, in germline configuration, all of the V, D and Jregions and intervening sequences from a human. Optionally, non-functional V and/or D and/or Jgene segments are omitted. For example, VH which are inverted or are pseudogenes may be omitted.

In one aspect 800-1000kb of the human IgH VDJ region is inserted into the non-human vertebrate IgH locus, and in one aspect a 940, 950 or 960 kb fragment is inserted. Suitably this includes bases 105,400,051 to 106,368,585 from human chromosome 14 (all coordinates refer to NCBI36 for the human genome, ENSEMBL Release 54 and NCBIM37 for the mouse genome, relating to mouse strain C57BL/6J).

In one aspect the inserted IgH human fragment consists of bases 105,400,051 to 106,368,585 from chromosome 14. In one aspect the inserted human heavy chain DNA, such as DNA consisting of bases 105,400,051 to 106,368,585 from chromosome 14, is inserted into mouse chromosome 12 between the end of the mouse J4 region and the Eµ region, suitably between coordinates 114,667,091 and 114,665,190, suitably at coordinate 114,667,091.

In one aspect the inserted human kappa VJregion comprises, in germline configuration, all of the V and J regions and intervening sequences from a human. Optionally, non-functional V and/or Jgene segments are omitted.

Suitably this includes bases 88,940,356 to 89,857,000 from human chromosome 2, suitably approximately 917kb. In a further aspect the light chain VJinsert may comprise only the proximal clusters of V segments and Jsegments. Such an insert would be of approximately 473 kb.

In one aspect the human light chain kappa DNA, such as the human IgK fragment of bases 88,940,356 to 89,857,000 from human chromosome 2, is suitably inserted into mouse chromosome 6 between coordinates 70,673,899 and 70,675,515, suitably at position 70,674,734.

In one aspect the human lambda VJregion comprises, in germline configuration, all of the V and J regions and intervening sequencesfrom a human. Suitably this includes analogous bases to those selected for the kappa fragment, from human chromosome 2. Optionally, non-functional V and/or Jgene segments are omitted.

All specific human antibody fragments described herein may vary in length, and may for example be longer or shorter than defined as above, such as 500 bases, 1KB, 2K, 3K, 4K, 5KB, 10 KB, 20KB, 30KB, 40KB or 50KB or more, which suitably comprise all or part of the human V(D)Jregion, whilst preferably retaining the requirement for the final insert to comprise human genetic material encoding the complete heavy chain region and light chain region, as appropriate, as described herein.

In one aspect the 3' end of the last inserted human antibody sequence, generally the last human J sequence, is inserted less than 2kb, preferably less than 1KB from the human/non-human vertebrate (eg; human/mouse or human/rat) join region.

Optionally, the genome is homozygous at the heavy chain locus and one, or both of Igλ and Igκ loci.

In another aspect the genome may be heterozygous at one or more of the light chain antibody loci, such as heterozygous for DNA encoding a chimaeric antibody chain and native (host cell) antibody chain. In one aspect the genome may be heterorygous for DNA capable of encoding 2 different antibody chains encoded by immunoglobulin transgenes as disclosed herein, for example, comprising 2 different chimaeric heavy chains or 2 different chimaeric light chains.

In one aspect in any configuration of the disclosure, the genome of the vertebrate has been modified to prevent or reduce the expression of fully-endogenous antibody. Examples of suitable techniques for doing this can be found in WO2011004192, US7501552, US6673986, U96130364, WO2009/076464, EP1399559 and US6586251. In one aspect, the non-human vertebrate VDJ region of the endogenous heavy chain immunoglobulin locus, and optionally VJregion of the endogenous light chain immunoglobulin loci (lambda and/or kappa loci), have been inactivated. For example, all or part of the non-human vertebrate VDJregion is inactivated by inversion in the endogenous heavy chain immunoglobulin locus of the mammal, optionally with the inverted region being moved upstream or downstream of the endogenous Ig locus. For example, all or part of the non-human vertebrate VJregion is inactivated by inversion in the endogenous kappa chain immunoglobulin locus of the mammal, optionally with the inverted region being moved upstream or downstream of the endogenous Ig locus. For example, all or part of the non-human vertebrate VJregion is inactivated by inversion in the endogenous lambda chain immunoglobulin locusof the mammal, optionally with the inverted region being moved upstream or downstream of the endogenous Ig locus. In one aspect the endogenous heavy chain locus is inactivated in this way as is one or both of the endogenous kappa and lambda loci.

Additionally or alternatively, the vertebrate has been generated in a genetic background which prevents the production of mature host B and T lymphocytes, optionally a RAG-1-deficient and/or RAG-2 deficient background. See US5859301 for techniques of generating RAG-1 deficient animals.

In one aspect in any configuration of the disclosure, the human V, Jand optional D regions are provided by all or part of the human IgH locus; optionally wherein said all or part of the IgH locus includes substantially the full human repertoire of IgH V, D and Jregions and intervening sequences. A suitable part of the human IgH locus is disclosed in WO2011004192. In one aspect, the human IgH part includes (or optionally consistsof) bases 105,400,051 to 106,368,585 from human chromosome 14 (coordinates from NCBI36). Additionally or alternatively, optionally wherein the vertebrate is a mouse or the cell is a mouse cell, the human V, Jand optional D regions are inserted into mouse chromosome 12 at a position corresponding to a position between coordinates 114,667,091 and 114,665,190, optionally at coordinate 114,667,091 (coordinatesfrom NCBIM37, relating to mouse strain C57BL/6J).

In one aspect of any configuration of a vertebrate or cell (line) disclosed herein when the vertebrate is a mouse, (i) each transgenic heavy chain locus of the mouse genome comprises a constant region comprising a mouse or rat Sµ switch and optionally a mouse Cµ region. For example the constant region is provided by the constant region endogenous to the mouse (mouse cell), eg, by inserting human V(D)J region sequences into operable linkage with the endogenous constant region of a mouse genome or mouse cell genome.

In one aspect of any configuration of a vertebrate or cell (line) disclosed herein when the Vertebrate is a rat, (i) each transgenic heavy chain locus of the rat genome comprises a constant region comprising a mouse or rat Sµ switch and optionally a rat Cµ region. For example the constant region is provided by the constant region endogenous to the rat, eg, by inserting human V(D)J region sequences into operable linkage with the endogenous constant region of a rat genome or rat cell genome.

In one aspect of any configuration of a vertebrate or cell (line) disclosed herein the genome comprises a lambda antibody transgene comprising all or part of the human Igλ locus including at least one human Jλ region and at least one human Cλ region, optionally C_{λ}6 and/or C_{λ}7. Optionally, the transgene comprisesa plurality of human Jλ regions, optionally two or more of J_{λ}1, J_{λ}2, J_{λ}6 and J_{λ}7, optionally all of J_{λ}1, J_{λ}2, J_{λ}6 and J_{λ}7. The human lambda immunoglobulin locus com prises a unique gene architecture composed of serial J-C clusters. In order to take advantage of this feature, the disclosure in optional aspects employs one or more such human J-C clusters inoperable linkage with the constant region in the transgene, eg, where the constant region is endogenous to the non-human vertebrate or non-human vertebrate cell (line). Thus, optionally the transgene comprises at least one human J_{λ}-C_{λ} cluster, optionally at least J_{λ}7-C_{λ}7. The construction of such transgenes is facilitated by being able to use all or part of the human lambda locus such that the transgene comprises one or more J-C clusters in germline configuration, advantageously also including intervening sequences between clusters and/or between adjacent Jand Cregions in the human locus. This preserves any regulatory elements within the intervening sequences which may be involved in VJ and/or JC recombination and which may be recognised by AID (activation-induced deaminase) or AID homologues.

Where endogenous regulatory elements are involved in CSR (class-switch recombination) in the non-human vertebrate, these can be preserved by including in the transgene a constant region that is endogenousto the non-human vertebrate. In the first configuration of the disclosure, one can match this by using an AID or AID homologue that is endogenousto the vertebrate or a functional mutant thereof. Such design elements are advantageous for maximising the enzymatic spectrum for SHM (somatic hypermutation) and/or CSRand thus for maximising the potential for antibody diversity.

Optionally, the lambda transgene comprises a human Eλ enhancer. Optionally, the kappa transgene comprises human Eκ enhancer. Optionally, the heavy chain transgene comprises a heavy chain human enhancer.

In one aspect of any configuration of the disclosure the constant region of the or each antibody transgene is endogenous to the non-human vertebrate or derived from such aconstant region. For example, the vertebrate isa mouse or the cell is a mouse cell and the constant region is endogenousto the mouse. For example, the vertebrate isa rat or the cell isa rat cell and the constant region is endogenous to the rat.

In one aspect of any configuration of the disclosure each heavy chain transgene comprises a plurality human IgH V regions, a plurality of human D regions and a plurality of human J regions, optionally substantially the full human repertoire of IgH V, D and Jregions.

In one aspect of any configuration of the disclosure, for the vertebrate:-
(i) each heavy chain transgene comprises substantially the full human repertoire of IgH V, D and Jregions; and
(ii) the vertebrate genome comprises substantially the full human repertoire of Igκ V and J regions and/or substantially the full human repertoire of Igλ V and J regions.

An aspect provides a B-cell, hybridoma or a stem cell, optionally an embryonic stem cell or haematopoietic stem cell, derived from a vertebrate according to any configuration of the disclosure. In one aspect, the cell is a BALB/c, JM8 or AB2.1 or AB2.2 embryonic stem cell (see discussion of suitable cells, and in particular JM8 and AB2.1 cells, in WO2011004192.

In one aspect the EScell isderived from the mouse BALB/c, C57BU6N, C57BL/6J, 129S5 or 129Sv strain.

In one aspect the non-human vertebrate is a rodent, suitably a mouse, and cells (cell lines) of the disclosure, are rodent cells or ES cells, suitably mouse ES cells.

The ES cells disclosed herein can be used to generate animals using techniques well known in the art, which comprise injection of the ES cell into a blastocyst followed by implantation of chimaeric blastocystys into females to produce offspring which can be bred and selected for homozygous recombinants having the required insertion. In one aspect the disclosure relates to a transgenic animal comprised of ES cell-derived tissue and host embryo derived tissue. In one aspect the disclosure relates to genetically-altered subsequent generation animals, which include animals having a homozygous recombinants for the VDJand/or VJ regions.

An aspect provides a method of isolating an antibody or nucleotide sequence encoding said antibody, the method comprising
(a) immunising (see e.g. Harlow, E & Lane, D. 1998, 5th edition, Antibodies: A Laboratory M anual, Cold Spring Harbor Lab. Press, Plainview, NY; and Pasqualini and Arap, Proceedings of the National Academy of Sciences (2004) 101:257-259) a vertebrate according to any configuration or aspect as disclosed herein with a human target antigen such that the vertebrate produces antibodies; and
(b) isolating from the vertebrate an antibody that specifically binds to said antigen and/or a nucleotide sequence encoding at least the heavy and/or the light chain variable regions of said antibody;
optionally wherein the variable regions of said antibody are subsequently joined to a human constant region. Such joining can be effected by techniques readily available in the art, such as using conventional recombinant DNA and RNA technology as will be apparent to the skilled person. See e.g. Sambrook, Jand Russell, D. (2001,3'd edition) Molecular Cloning: A Laboratory Manual (Cold Spring Harbor Lab. Press, Plainview, NY).

Suitably an immunogenic amount of the human epitope or target antigen is delivered. The disclosure also relates to a method for detecting a human epitope or target antigen comprising detecting a test antibody produced as above with a secondary detection agent which recognises a portion of that antibody.

Isolation of the antibody in step (b) can be carried out using conventional antibody selection techniques, eg, panning for antibodies against antigen that has been immobilised on a solid support, optionally with iterative rounds at increasing stringency, as will be readily apparent to the skilled person.

Asafurther optional step, after step (b) the amino acid sequence of the heavy and/or the light chain variable regions of the antibody are mutated to improve affinity for binding to said antigen. Mutation can be generated by conventional techniques as will be readily apparent to the skilled person, eg, by error-prone PCR. Affinity can be determined by conventional techniques as will be readily apparent to the skilled person, eg, by surface plasmon resonance, eg, using Biacore^{™}.

Additionally or alternatively, as a further optional step, after step (b) the amino acid sequence of the heavy and/or the light chain variable regions of a test antibody are mutated to improve one or more biophysical characteristics of the antibody, eg, one or more of melting temperature, solution state (monomer or dimer), stability and expression (eg, in CHO or *E coli*).

Disclosed herein is an antibody, optionally for use in medicine, eg, for treating and/or preventing a medical condition or disease in a patient, eg, a human.

An aspect provides a nucleotide sequence encoding an antibody as disclosed herein, optionally wherein the nucleotide sequence is part of a vector. Suitable vectors will be readily apparent to the skilled person, eg, aconventional antibody expression vector comprising the nucleotide sequence together in operable linkage with one or more expression control elements.

An aspect provides a pharmaceutical composition comprising an antibody as disclosed herein and a diluent, excipient or carrier, optionally wherein the composition iscontained in an IV container (eg, and IV bag) or a container connected to an IV syringe.

An aspect provides the use of an antibody disclosed herein in the manufacture of a medicament for the treatment and/or prophylaxis of a disease or condition in a patient, eg a human.

Disclosed herein are humanised antibodies and antibody chains produced or assayed as disclosed herein, both in chimaeric and fully humanised form, and use of said antibodies in medicine. Disclosed herein is a pharmaceutical composition comprising such an antibody and a pharmaceutically acceptable carrier or other excipient.

Antibody chains containing human sequences, such as chimaeric human-non human antibody chains, are considered humanised herein by virtue of the presence of the human protein coding regions region. Fully human antibodies may be produced starting from DNA encoding a chimaeric antibody chain as disclosed herein using standard techniques.

Methodsfor the generation of both monoclonal and polyclonal antibodies are well known in the art, and the present disclosure relates to both polyclonal and monoclonal antibodies of chimaeric or fully humanised antibodies produced in response to antigen challenge in non human-vertebrates of the present disclosure.

In a yet further aspect, chimaeric antibodies or antibody chains generated in the present disclosure may be manipulated, suitably at the DNA level, to generate molecules with antibody-like properties or structure, such as a human variable region from a heavy or light chain absent a constant region, for example a domain antibody; or a human variable region with any constant region from either heavy or light chain from the same or different species; or a human variable region with a non-naturally occurring constant region; or human variable region together with any other fusion partner. The disclosure relates to all such chimaeric antibody derivatives derived from chimaeric antibodies identified, isolated or assayed as disclosed herein.

It will be understood that particular embodiments described herein are shown by way of illustration and not as limitations of the invention. The principal features of this invention can be employed in various embodiments without departing from the scope of the invention as defined in the claims. Those skilled in the art will recognize, or be able to ascertain using no more than routine study, numerous equivalents to the specific procedures described herein. All publications and patent applications mentioned in the specification are indicative of the level of skill of those skilled in the art to which this invention pertains. The use of the word "a" or "an" when used in conjunction with the term "comprising" in the claims and/or the specification may mean "one," but it is also consistent with the meaning of "one or more," "at least one," and "one or more than one." The use of the term "or" in the claims is used to mean "and/or" unless explicitly indicated to refer to alternatives only or the alternatives are mutually exclusive, although the disclosure supports a definition that refers to only alternatives and "and/or." Throughout this application, the term "about" is used to indicate that a value includes the inherent variation of error for the feature in the context with which it is referred. The term "substantially" when referring to an amount, extent or feature (eg, "substantially identical" or "substantially the same") includes a disclosure of "identical" or "the same" respectively, and this provides basis for insertion of these precise terms into claims below.

As used in this specification and claim(s), the words"comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps.

The term "or combinations thereof" as used herein refers to all permutations and combinations of the listed items preceding the term. For example, "A, B, C, or combinations thereof is intended to include at least one of: A, B, C, AB, AC, BC, or ABC, and if order is important in a particular context, also BA, CA, CB, CBA, BCA, ACB, BAC, or CAB. Continuing with this example, expressly included are combinations that contain repeats of one or more item or term, such as BB, AAA, M B, BBC, AAABCCCC, CBBAAA, CABABB, and so forth. The skilled artisan will understand that typically there is no limit on the number of items or terms in any combination, unless otherwise apparent from the context.

Any part of this disclosure may be read in combination with any other part of the disclosure, unless otherwise apparent from the context.

All of the compositions and methods disclosed and the methods as claimed herein can be made and executed without undue experimentation in light of the present disclosure. While thecompositionsdisclosed herein and methods of this invention have been described in terms of preferred embodiments, it will be apparent to those of skill in the art that variations may be applied to the compositions and/or methods and in the stepsor in the sequence of stepsof the method described herein without departing from the concept of the invention. All such similar substitutes and modifications apparent to those skilled in the art are deemed to be within the concept of the invention as defined by the appended claims.

The present invention is described in more detail in the following non limiting exemplification.

### EXAM PLES

The following examples will be useful for demonstrating the present invention.

### INACTIVATION OF ENDOGENOUSIGH GENES AND MAINTENANCE OF ADAM 6 FUNCTION

### Example 1 : Translocation

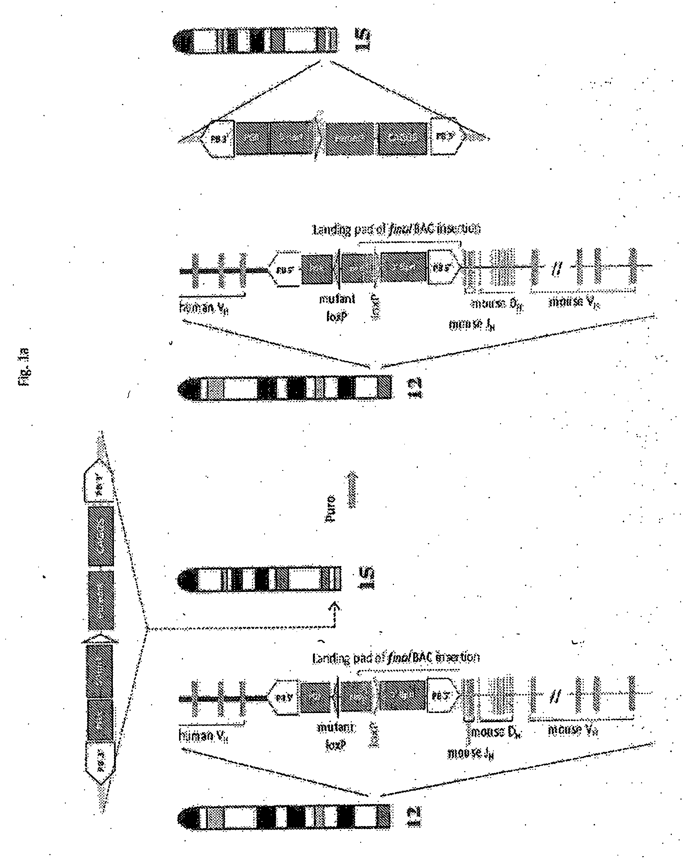

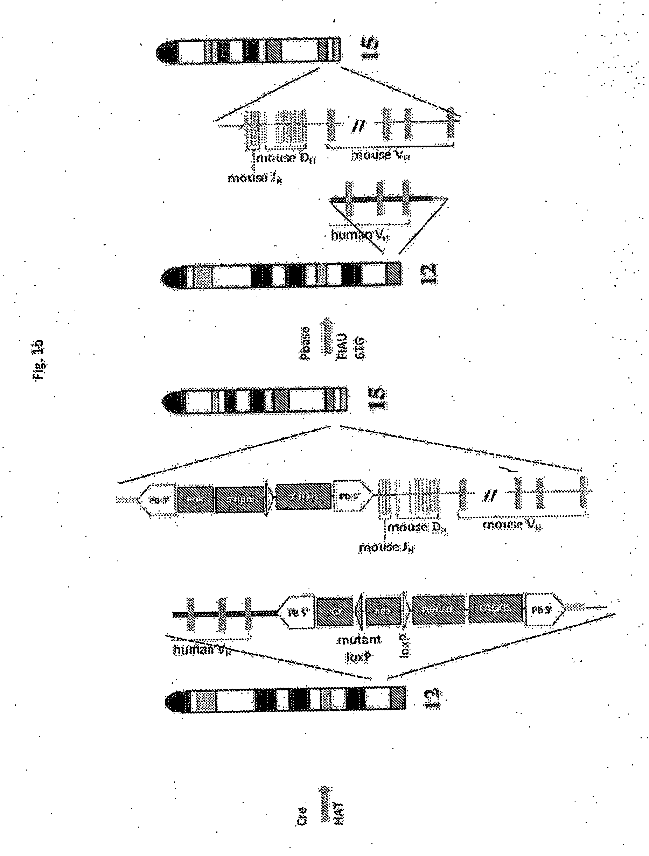

Reference is made to Fig 1a where chromosome 12 is shown harbouring a transgenic heavy chain locus. In the figure, the inserted human V_{H} gene segments are shown (but for clarity the human D and J_{H}, the mouse Emu enhancer and other J-C intronic elements, and also the constant region are not shown, but these lie downstream of the human V_{H} gene segments (ie, to the left of the V_{H}). Also shown isa IoxP site on chromosome 12 between the human V_{H} and the mouse VDJregion (in this case the IoxP being provided by a "landing pad"; see, eg, WO2011004192. A cassette, carrying a IoxP site in the same direction to the IoxP site in the landing pad, istargeted at the telomere region of a different chromosome from chromosome 12; in this case targeting is to chromosome 15 as shown in Fig 1a. A vector carrying a Cre recombinase gene is introduced into the cell. Following induction of Cre recombinase expression, the regions between the IoxP sites and the telomeres are exchanged, which results in separation of the endogenous mouse V_{H}, D and J_{H} gene segments away from their enhancer and C region (Fig 1 b) and thus inactivation of endogenous heavy chain. In this method, the upstream and downstream genome sequence of Adam6a and Adam6b which includes the associated regulatory elements of these two genes, is still retained intact. Thus functional, endogenous Adam6 genes are retained - in thiscase on chromosome 15 (Fig. 1b).

### Example 2: Deletion & Insertion of Adam 6 Genes

### Generation of Transgenic Antibody-Generating Mouse

A transgenic mouse is generated using ES cell technology and genetic manipulation to introduce human antibody heavy chain and kappa chain V, D and J segments operatively connected directly 5' of endogenous mouse heavy and kappa constant regions respectively. Mouse mu switch and mu constant and gamma regions are provided in the heavy chain transgenic locus thus produced. Endogenous, mouse heavy chain and kappa chain expression are inactivated; mouse lambda chain expression is typically 5% or less so inactivation is optional. The human antibody gene segments are introduced into a mouse ES cell using homologous recombination and/or recombinase mediated cassette exchange'(RMCE) as is known in the art. Human DNA can be manipulated using BAC and recombineering technology as known in the art. BACs containing human antibody gene DNA is obtainable from Invitrogen. A suitable EScell is a 129, AB2.1 or AB2.2 cell (obtainable from Baylor College of Medicine).

The transgenic ES cells are then implanted into a blastocyst from a foster mouse mother (eg, a 129 or C57BL/6N mouse strain). Heavy chain and kappa chain lines can be produced and crossed to provide an antibody-generating mouse bearing homozygous transgenic heavy and kappachainswith human variable regions (HK mouse).

Using a similar protocol, a lambda chain line is produced and by crossing a HKL mouse is generated bearing homozygous transgenic heavy, lambda and kappa chains with human variable regions.

Further guidance is disclosed in WO2011004192, US7501552, US6673986, US6130364, WO2009/076464 and US6586251.

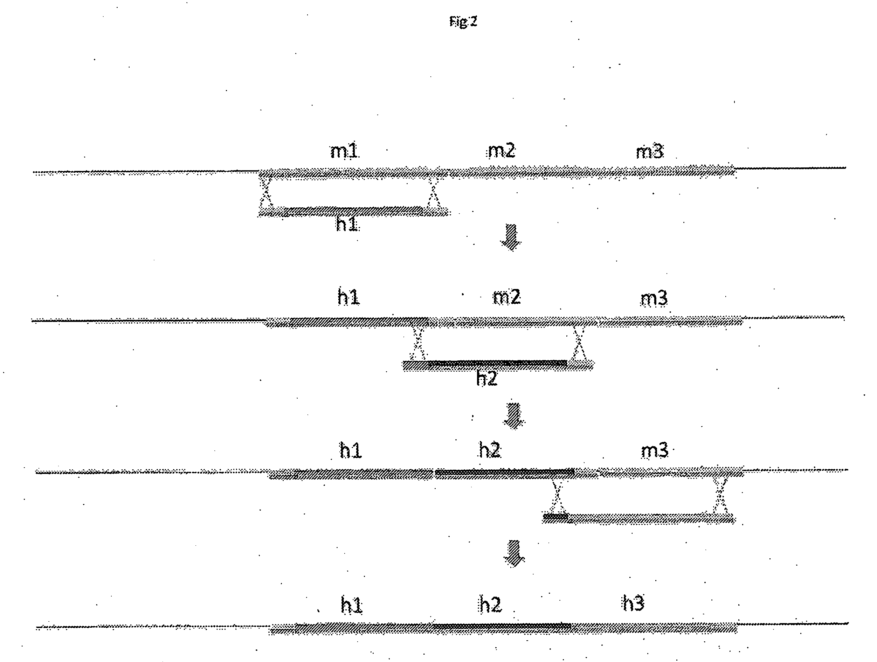

In order to introduce human heavy chain gene segments by homologous recombination, one or more BACs are generated using standard techniques such as recombineering. A large DNA targeting vector containing human genomic IGH gene segments (V_{H}s, Ds and J_{H}s), a selection marker and two flanking recombination arms(5' and 3') homologous to the endogenous IGH sequence is constructed by BACmodification (Fig 2; h1 = first BACcontaining human gene segments; m1 = homologous region of the mouse VDJregion; and so on for h1, h2, m2 and m3). The large targeting vector is introduced into mouse ES cells by electroporation. The targeted ES cells are selected by drugs or other marker sorting for the selection marker as is conventional. The correct targeting by homologous recombination is further confirmed by either quantitative or qualitative PCR-based methods. The correctly targeted locus results in replacement of endogenous genomic DNA flanked by those two homologous recombination arms, in which this section of the endogenous locus is replaced with the human genomic IGH gene segments and a selection marker.

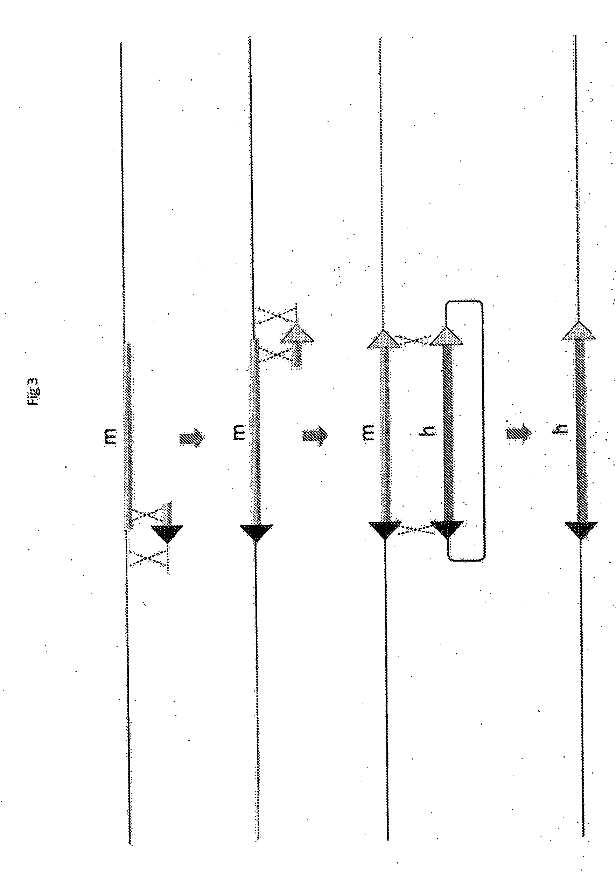

The human antibody heavy chain gene segments ("h" in Fig 3) can also be inserted using standard recombinase-mediated genomic replacement (Fig. 3). In such an approach, one IoxP site and a mutant IoxP site (such as Iox511) are sequentially targeted into the mouse IGH locus. A large DNA targeting vector containing human genomic IGH gene segments (V_{H}s, Ds and J_{H}s) and a selection marker, flanked by one IoxP site and another copy of the mutant IoxP is constructed by BAC modification. The large targeting vector is co-electroporated with a Cre-expressing vector into ES cells. The correct targeting is further confirmed by either quantitative or qualitative PCR-based methods. The correctly targeted locus results in the replacement of endogenous antibody locus genomic DNA flanked by those two lox sites, in which this section of the endogenous locus ("m" in Fig 3) is replaced with the human genomic IGH gene segments and a selection marker. In this process, endogenous Adam 6 genes are also deleted.

During these two replacement processes (replacement by homolgous recombination or RMGR), the endogenous mouse Adam6 genes between the V_{H}5-1 and D1-1 gene segments are deleted. The genomic DNAs containing the Adam6 exons (Adam 6a-2507 bp; Adam 6b-2271 bp) as well as at least 5 kb upstream and 5 kb downstream sequences for each of them are inserted into mouse genome by either targeted or random insertion in ES cells or zygotes to rescue the male fertility of such Adam6-deleted mice as per Example 3.

### Example 3: Approaches to insert Adam 6 genes into genome after endogenous IGH deletion

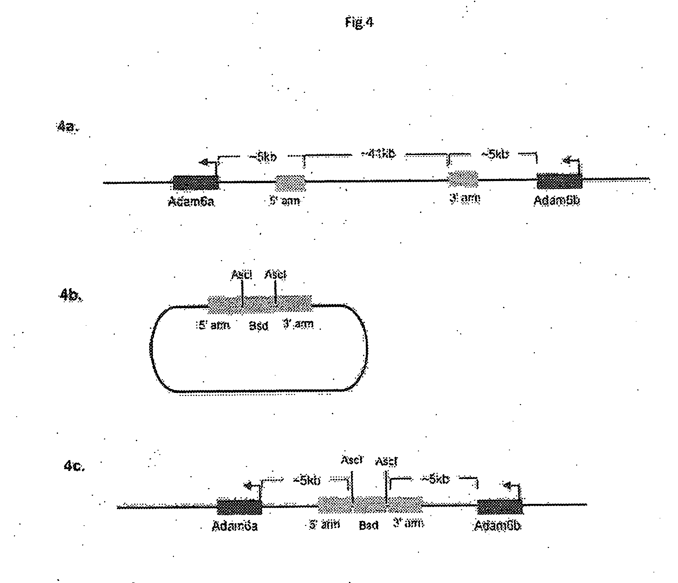

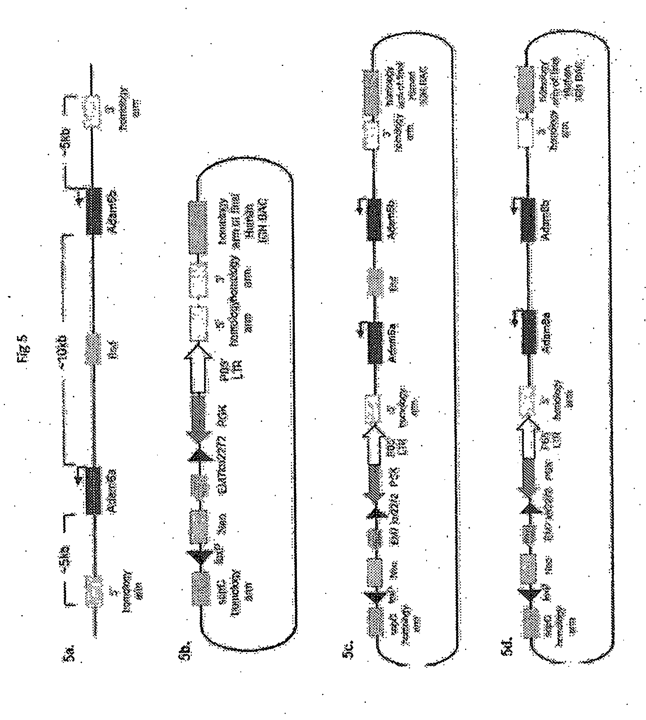

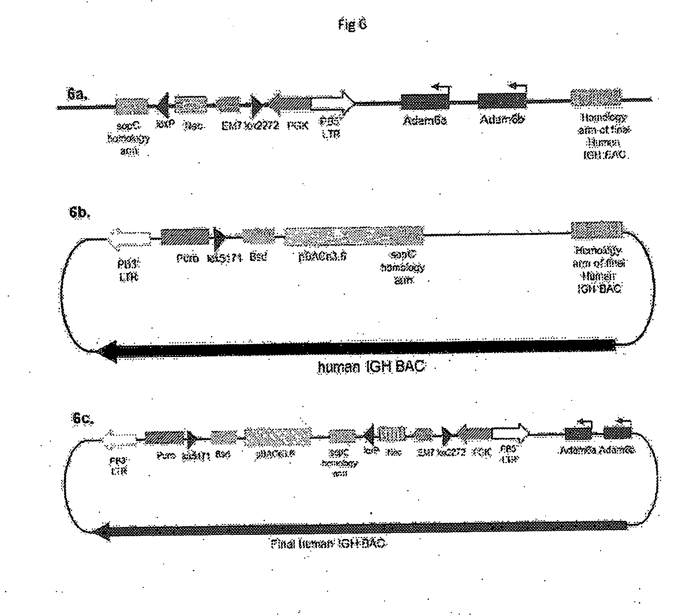

The mouse Adam6a (Chromosome 12: coordinates 114777119-114789625) and Adam6b (Chromosome 12: coordinates 114722756-114735229) genom ic DNA is retrieved from a bacterial artificial chromosome (BAC), RP23-393F3 (Invitrogen). The ES-cell targeting vector is generated by the following steps.
1. The sequence between mouse Adam6a and Adam6b is deleted by a positive selection marker cassette.
   a. 5' arm which is located at ~ 5kb upstream of Adam6a and 3' arm which is located at ~5kb downstream of Adam6b gene are created by PCR using RP23-393F3 as a template. Both homology arms are between 200bp to 300bp, then the two homology arms are cloned into a plasmid based on pBlueScript II SK(+) and that contains a positive selection marker Blasticidin (Bsd) which flanked by two AscI sites, to build a deletion vector (Fig. 4a and 4b).
      5' Arm: 3' Arm:
   b. The sequence between mouse Adam6a and Adam6b is deleted by targeting the Bsd cassette to RP23-393F3 (Fig. 4c). In such a recombineered product, ~5kb upstream sequence of and ~5kb downstream sequence of Adam6b and Adam6a respectively are still kept to maintain their specific regulation of expression in mouse cells.
2. Mouse Adam6a and Adam6b are retrieved to the 5' modifying vector of the IGH BAC by homologous recombination.
   a. 5' homology arm located at ~ 5kb downstream of Adam6a or 3' homology arm located at ~5kb upstream of Adam6b gene are created by PCR using RP23-393F3 as a template (Fig. 5a).
      5' Arm: 3' Arm:
   b. Two homology arms are cloned into the 5' modification vector (the vector being based on pBR322). This 5' modification vector has the gene sopC (required to ensure that each daughter cell getsacopy of the plasmid), homology arm, IoxP, Neo cassette, IoxP 2272, PGKpromoter, PB5' LTRand the homology arm: of the final Human IGH BAC (Fig 5b).
   c. BACsequence from ~ 5kb downstream of Adam6a gene to ~5kb upstream of Adam6b gene is retrieved into the 5' modifying vector of the IGH BAC by standard recombineering (Fig.5c).
   d. After retrieving, the targeting vector is constructed by removing the Bsd gene through AscI digestion and self-ligation (Fig. 5d).
3. The retrieved Adam6a & Adam6b along with the 5'modifying cassette (Fig. 6a) is targeted into the IGH BAC (Fig 6b) through standard recombineering to generate the final IGH BAC (Fig. 6c).

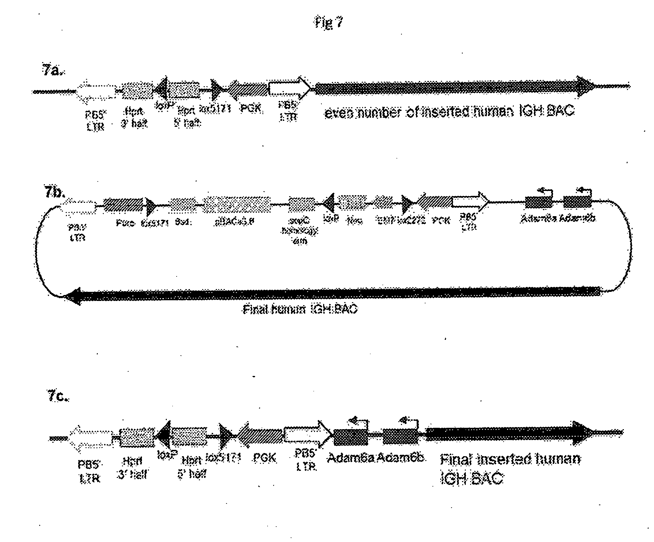

Mouse Adam6a and Adam6b along with the final human IGH BAC are inserted into mouse genome by recombinase-mediated cassette exchange (RMCE), as shown in figure 7a to 7c and as described in WO2011004192. The inserted Adam6a and Adam6b can rescue the Adam6-deficient phenotype as per the present disclosure.

### Example 4: Fertile M ice & Progeny Comprising ADAM 6 Genes

Using recombineering and ES cell genomic manipulation, mouse AB2.1 embryonic stem cell genomes were engineered to insert varying repertoires of human variable region gene segments upstream of endogenous mouse constant regions in endogenous IgH loci to functionally replace endogenous mouse variable regions. The endogenous VDJ region was deleted from the IgH loci, thereby removing the ADAM 6a and ADAM 6b genes from the loci. Expressible mouse ADAM6a and ADAM6b genes with wild-type promoters were inserted upstream of the IgH locus on mouse chromosome 12. Progeny mice were developed that were heterozygous for the IgH transgene (ie, having genomes with one copy of the transgenic IgH locus and with the other IgH locus rendered non-functional). Fertile heterozygous mice were obtained and bred together to produce homozygous progeny. These progeny were homozygous for the IgH transgene having the ADAM 6 deletion and also homozygous for the inserted mouse ADAM 6a and 6b genes. Moreover, we obtained fertile male and female homozygotes that were able to breed and produce progeny. A summary is provided below.

Three different homozygous lines were produced: IgH 1 mice; IgH 2 mice and IgH3 mice. These mice were homozygous for deletion of ADAM6 genesfrom the endogenousmouse IgH locus, homozygous for insertion of mouse ADAM 6a and ADAM6b genes on chromosome 12 (upstream of the IgH locus) and homozygous for a heavy chain transgene as follows.
IgH 1 transgene:
   comprises human heavy gene segments V_{H}2-5, V_{H}7-4-1, V_{H}4-4, V_{H}1-3, V_{H}1-2, V_{H}6-1, D1-1, D2-2, D3-9, D3-10, D4-11, D5-12, D6-13, D1-14, D2-15, D3-16, D4-17, D5-18, D6-19, D1-20, D2-21, D3-22, D4-23, D5-24, D6-25, D1-26, D7-27, J_{H}1, J_{H}2, J_{H}3, J_{H}4, J_{H}5 and J_{H}6.
IgH 2 transgene:
   comprises human heavy gene segments V_{H}3-13, V_{H}3-11, V_{H}3-9, V_{H}1-8, V_{H}3-7, V_{H}2-5, V_{H}7-4-1, V_{H}4-4, V_{H}1-3, V_{H}1-2, V_{H}6-1, D1-1, D2-2, D3-9, D3-10, D4-11, D5-12, D6-13, D1-14, D2-15, D3-16, D4-17, D5-18, D6-19, D1-20, D2-21, D3-22, D4-23, D5-24, D6-25, D1-26, D7-27, J_{H}1, J_{H}2, J_{H}3, J_{H}4, J_{H}5 and J_{H}6.
IgH 3 transgene:
   comprises human heavy gene segments VH2-26, VH1-24, VH3-23, VH3-21, VH3-20, VH1-18, VH3-15, V_{H}3-13, V_{H}3-11, V_{H}3-9, V_{H}1-8, V_{H}3-7, V_{H}2-5, V_{H}7-4-1, V_{H}4-4, V_{H}1-3, V_{H}1-2, V_{H}6-1, D1-1, D2-2, D3-9, D3-10, D4-11, D5-12, D6-13, D1-14, D2-15, D3-16, D4-17, D5-18, D6-19, D1-20, D2-21, D3-22, D4-23, D5-24, D6-25, D1-26, D7-27, J_{H}1, J_{H}2, J_{H}3, J_{H}4, J_{H}5 and J_{H}6.

In order to assess whether or not mice were capable of breeding, we set up various test crosses between homozygote males and fertile female mice asfollows:-

| | | NUMBER OF LITTERS | TOTAL NUMBER OF PROGENY | AVERAGE NUMBER OF PROGENY |
|---|---|---|---|---|
| Control Crosses | Wild-type (WT) male x fertile female | 21 | 153 | 7.3 ± 2.3 |
| IgH 1 Test Crosses | Homozygous IgH 1 male x fertile female | 17 | 132 | 7.6 ± 2.5 |
| IgH 2 Test Crosses | Homozygous IgH 2 male x fertile female | 5 | 35 | 7.0 ± 5.2 |
| IgH 3 Test Crosses | Homozygous IgH 3 male x fertile female | 22 | 162 | 7.4 ± 3.2 |

Thus, we were able to show the production of fertile male and female mice that were either heterozygous or homozygous for the heavy chain transgene and the deletion of endogenous VDJ. Furthermore, these mice were either heterozygous or homozygous for inserted ADAM 6.

In addition, the litter size of test crosses is not significantly changed (7.7 ± 3.5 mice as an average for all test crosses) from that of matings using wild-type males (8.1 ±3.1 mice).

In further experiments, we immunised homozygoustest mice with human antigens and observed a specific immune response. Both prime-boost and RIM MSimmunisation protocols were used. We isolated antigen-specific B-cells and antibodies from such mice as well as nucleic acid sequences encoding such antibodies and their chains and variable regions. Furthermore, we successfully produced hybridomasfrom such antigen-specific B-cells.

## Claims

1. A method of preparing a pharmaceutical composition comprising an antibody and a diluent, excipient or carrier, the method comprising
(a) immunising a fertile rodent which is a mouse or rat with a human target antigen such that the mouse or rat produces antibodies; and
(b) isolating from the mouse or rat an antibody that specifically binds to said antigen and/or isolating a nucleotide sequence encoding at least the heavy and/or the light chain variable regions of such an antibody; and
optionally subsequently joining the variable regions of said antibody to a human constant region,
wherein the fertile mouse or rat is optionally male, and is homozygous for a transgenic antibody heavy chain locus, the mouse or rat having a genome that
(a) comprises each heavy chain locus on a respective copy of a first chromosome; and
(b) is inactivated for endogenous antibody heavy chain expression;
wherein each first chromosome of the genome comprises
(c) a transgenic antibody heavy chain locus comprising one or more human VH gene segments, one or more human D gene segments and one or more human JH gene segments operably connected upstream of a mouse or rat heavy chain constant region, optionally Cmu and/or Cgamma;
(d) a deletion of all or part of the endogenous heavy chain VDJ region of said chromosome to inactivate endogenous antibody heavy chain expression, wherein the deletion includes ADAM6;
wherein the genome comprises
(e) an insertion of one or more expressible ADAM6 genes;
wherein the genome comprises inserted expressible mouse ADAM6a and ADAM6b genes; or wherein the genome comprises an inserted expressible rat ADAM6 gene, wherein each ADAM6 nucleic acid sequence is inserted so that it is operably connected to a promoter for expression,
and wherein the composition is contained in an intravenous (IV) bag or a container connected to an IV syringe.

2. The method of claim 1, wherein a prime and boost protocol is used for the immunisation or wherein a RIMMS protocol is used for the immunisation.

3. The method of claim 1 or 2, wherein the rodent is a mouse, and wherein the mouse genome comprises a transgenic antibody heavy chain locus on a copy of chromosome 12.

4. The method of claim 1 or 2 wherein the rodent is a rat, and wherein the rat genome comprises a transgenic antibody heavy chain locus on a copy of chromosome 6.

5. The method of claim 3 or claim 4, wherein each inserted ADAM6 gene is on a
(i) chromosome 12 wherein the animal is a mouse; or
(ii) chromosome 6 wherein the animal is a rat.

6. The method of any preceding claim wherein an ADAM6 gene is inserted
(i) within one or both transgenic heavy chain loci or
(ii) within 20Mb of one or both transgenic heavy chain loci.

7. The method of any preceding claim, wherein the human gene segments replace all or part of the endogenous VDJ region in each heavy chain locus.

8. The method of any preceding claim, wherein the genome is homozygous for each inserted ADAM6 gene; optionally wherein the genome comprises more than two copies of ADAM6 genes selected from rat ADAM6, mouse ADAM6a and mouse ADAM6b genes.

9. The method of any preceding claim, wherein the genome comprises expressible ADAM6 that is inserted from a targeting vector, eg, a BAC vector.

10. The method of any preceding claim, wherein the expressible ADAM6 gene(s) is a rat ADAM6 nucleotide sequence operably connected to an endogenous mouse ADAM6 promoter or a mouse ADAM6 nucleotide sequence operably connected to an endogenous rat ADAM6 promoter.

11. The method of any preceding claim, wherein the genome comprises an inserted human VDJ region that includes bases 105,400,051 to 106,368,585 from human chromosome 14.

12. The method of any preceding claim, wherein the genome is homozygous at the Ig heavy chain, Igλ and Igκ loci.

13. The method of any preceding claim, wherein the genome comprises:
(a) Heavy chain loci, each comprising one or more human heavy chain V gene segments, one or more human heavy chain D gene segments and one or more human heavy chain JH gene segments upstream of an endogenous mouse or rat constant region or a human constant region, eg, Cmu and/or Cgamma;
(b) A kappa light chain locus, optionally in homozygous state, comprising one or more human kappa chain V gene segments, and one or more human kappa chain Jκ gene segments upstream of an endogenous mouse or rat kappa constant region; and optionally
(c) A lambda light chain locus, optionally in homozygous state, comprising one or more human lambda chain V gene segments, and one or more human lambda chain Jλ gene segments upstream of a lambda constant region; and
(d) Wherein the mouse or rat is capable of producing chimaeric antibodies following rearrangement of said loci and immunisation with an antigen.

14. The method of any preceding claim, wherein the rodent is a mouse and the genome comprises an insertion of human light chain kappa VJ targeted into mouse chromosome 6 between coordinates 70,673,899 and 70,675,515, suitably at position 70,674,734, or an equivalent position in the lambda mouse locus on chromosome 16.

15. The method of any preceding claim, wherein human VH which is naturally inverted or pseudogene VH is omitted from the genome.

## Patentansprüche

1. Ein Verfahren zur Zubereitung einer pharmazeutischen Zusammensetzung, die einen Antikörper und ein Verdünnungsmittel, einen Hilfsstoff oder einen Träger umfasst, wobei das Verfahren umfasst
(a) Immunisieren eines fertilen Nagetiers, das eine Maus oder eine Ratte ist, mit einem humanen Zielantigen, so dass die Maus oder Ratte Antikörper produziert; und
(b) Isolieren eines Antikörpers von der Maus oder Ratte, der spezifisch an das besagte Antigen bindet und/oder Isolieren einer Nukleotidsequenz, die zumindest für die schwerkettigen und/oder leichtkettigen variablen Regionen eines solchen Antikörpers kodiert; und
optional, danach das Verbinden der variablen Regionen des besagten Antikörpers mit einer humanen Konstantregion, wobei die fertile Maus oder Ratte optional männlich und homozygot für einen transgenen schwerkettigen Antikörperlocus ist, wobei die Maus oder die Ratte ein Genom aufweisen, das
(a) jeden schwerkettigen Locus auf einem jeweiligen Exemplar eines ersten Chromosoms umfasst; und
(b) inaktiviert ist für die endogene schwerkettige Antikörperexpression; wobei jedes erste Chromosom des Genoms
(c) einen transgenen schwerkettigen Antikörperlocus, der ein oder mehrere humane VH-Gensegmente, ein oder mehrere humane D-Gensegmente und ein oder mehrere humane JH-Gensegmente umfasst, die funktionsfähig verbunden sind stromaufwärts einer schwerkettigen Konstantregion einer Maus oder Ratte, optional Cmu und/oder Cgamma;
(d) eine Deletion der gesamten oder eines Teils der endogenen schwerkettigen VDJ-Region des besagten Chromosoms umfasst, um die endogene schwerkettige Antikörperexpression zu inaktivieren, wobei die Deletion ADAM6 umfasst;
wobei das Genom
(e) eine Insertion von einem oder mehreren expressionsfähigen ADAM6-Genen umfasst;
wobei das Genom eingesetzte expressionsfähige ADAM6a- und ADAM6b-Gene der Maus umfasst; oder wobei das Genom ein eingesetztes expressionsfähiges ADAM6-Gen der Ratte umfasst, wobei jede ADAM6-Nukleinsäuresequenz so eingesetzt wird, so dass sie funktionsfähig mit einem Promoter zur Expression verbunden ist, und wobei die Zusammensetzung in einem intravenösen (IV-) Beutel oder einem Behältnis, das mit einer IV-Spritze verbunden ist, enthalten ist

2. Das Verfahren nach Anspruch 1, wobei ein Prime-and-Boost-Protokol für die Immunisierung verwendet wird oder wobei ein RIMMS-Protokoll für die Immunisierung verwendet wird.

3. Das Verfahren nach Anspruch 1 oder 2, wobei das Nagetier eine Maus ist und wobei das Maus-Genom einen schwerkettigen transgenen Antikörperlocus auf einem Exemplar des Chromosoms 12 umfasst.

4. Das Verfahren nach Anspruch 1 oder 2, wobei das Nagetier eine Ratte ist und wobei das Ratten-Genom einen schwerkettigen transgenen Antikörperlocus auf einem Exemplar des Chromosoms 6 umfasst.

5. Das Verfahren nach Anspruch 3 oder 4, wobei jedes eingesetzte ADAM6-Gen auf einem
(i) Chromosom 12 ist, wobei das Tier eine Maus ist; oder
(ii) Chromosom 6 ist, wobei das Tier eine Ratte ist.

6. Das Verfahren nach einem der vorhergehenden Ansprüche, wobei ein ADAM6-Gen eingesetzt wird
(i) innerhalb eines oder beider transgener schwerkettiger Loci oder
(ii) innerhalb 20 Mb eines oder beider transgener schwerkettiger Loci.

7. Das Verfahren nach einem der vorhergehenden Ansprüche, wobei die humanen Gensegmente das ganze oder einen Teil der endogenen VDJ-Region in jedem schwerkettigen Locus ersetzen.

8. Das Verfahren nach einem der vorhergehenden Ansprüche, wobei das Genom homozygot ist für jedes eingesetzte ADAM6-Gen; wobei optional das Genom mehr als zwei Exemplare der ADAM6-Gene umfasst, die aus dem ADAM6-Gen der Ratte, dem ADAM6a-Gen der Maus und dem ADAM6b-Gen der Maus ausgewählt sind.

9. Das Verfahren nach einem der vorhergehenden Ansprüche, wobei das Genom expressionsfähiges ADAM6 umfasst, das von einem Zielvektor eingesetzt ist, zum Beispiel von einem BAC-Vektor.

10. Das Verfahren nach einem der vorhergehenden Ansprüche, wobei das/die expressionsfähige ADAM6-Gen(e) eine ADAM6-Nukleotidsequenz der Ratte ist/sind, die funktionsfähig verbunden ist mit einem endogenen ADAM6-Promoter der Maus oder eine ADAM6-Nukleotidsequenz der Maus, die funktionsfähig verbunden ist mit einem endogenen ADAM6-Promoter der Ratte.

11. Das Verfahren nach einem der vorhergehenden Ansprüche, wobei das Genom eine eingesetzte humane VDJ-Region umfasst, die die Basen 105.400.051 bis 106.368.585 des humanen Chromosoms 14 umfasst.

12. Das Verfahren nach einem der vorhergehenden Ansprüche, wobei das Genom an den Loci der Ig-Schwerkette, Ig Lambda und Ig Kappa homozygot ist.

13. Das Verfahren nach einem der vorhergehenden Ansprüche, wobei das Genom umfasst:
(a) schwerkettige Loci, jeweils ein oder mehrere humane schwerkettige V-Gensegmente, ein oder mehrere humane schwerkettige D-Gensegmente und ein oder mehrere humane schwerkettige JH-Gensegmente stromaufwärts einer endogenen Konstantregion der Maus oder Ratte oder einer humanen Konstantregion, zum Beispiel Cmu und/oder Cgamma, umfassend;
(b) einen leichtkettigen Kappa-Locus, optional in einem homozygoten Zustand, umfassend ein oder mehrere humane V-Gensegmente der Kappa-Kette, und ein oder mehrere humane J-Kappa-Gensegmente der Kappa-Kette stromaufwärts einer endogenen Kappa-Konstantregion der Maus oder Ratte; und optional
(c) einen leichtkettigen Lambda-Locus, optional in einem homozygoten Zustand, umfassend ein oder mehrere humane V-Gensegmente der Lambda-Kette; und ein oder mehrere humane J-Lambda-Gensegmente der Lambda-Kette stromaufwärts einer Lambda-Konstantregion; und
(d) wobei die Maus oder Ratte in der Lage ist, nach einem Rearrangement der besagten Loci und einer Immunisierung mit einem Antigen chimäre Antikörper zu produzieren.

14. Das Verfahren nach einem der vorhergehenden Ansprüche, wobei das Nagetier ein Maus ist und das Genom eine Insertion einer humanen leichtkettigen Kappa-VJ umfasst, die auf das Maus-Chromosom 6 gerichtet ist zwischen den Koordinaten 70.673.899 und 70.675.515, geeigneterweise an der Position 70.674.734 oder einer äquivalenten Position im Lambda-Locus der Maus auf Chromosom 16.

15. Das Verfahren nach einem der vorhergehenden Ansprüche, wobei humanes VH, das natürlich invertiert ist, oder Pseudogen-VH aus dem Genom ausgelassen wird.

## Revendications

1. Procédé de préparation d'une composition pharmaceutique comprenant un anticorps et un diluant, un excipient ou un véhicule, le procédé comprenant les étapes consistant à :
(a) immuniser un rongeur fertile qui est une souris ou un rat avec un antigène cible humain de sorte que la souris ou le rat produise des anticorps ;
(b) isoler de la souris ou du rat un anticorps qui se lie spécifiquement audit antigène et/ou isoler une séquence nucléotidique codant pour au moins les régions variables à chaînes lourdes et/ou à chaînes légères d'un tel anticorps ; et
joindre ensuite éventuellement les régions variables dudit anticorps à une région humaine constante, dans lequel la souris ou la rat fertile est éventuellement un mâle et est homozygote pour un locus de chaîne lourde d'anticorps transgénique, la souris ou le rat ayant un génome qui :
(a) comprend chaque locus de chaîne lourde sur une copie respective d'un premier chromosome ; et
(b) est inactivé pour une expression de chaîne lourde d'anticorps endogène ;
dans lequel chaque premier chromosome du génome comprend :
(c) un locus de chaîne lourde d'anticorps transgénique comprenant un ou plusieurs segments de gène humain VH, un ou plusieurs segments de gène humain D et un ou plusieurs segments de gène humain JH raccordés en service en amont d'une région constante de chaîne lourde de souris ou de rat, éventuellement Cmu et/ou Cgamma ;
(d) une délétion de la totalité ou d'une partie de la région VDJ de chaîne lourde endogène dudit chromosome pour inactiver l'expression de chaîne lourde d'anticorps endogène, dans lequel la délétion comprend l'ADAM6 :
dans lequel le génome comprend :
(e) une insertion d'un ou plusieurs gènes ADAM6 expressibles ;
dans lequel le génome comprend des gènes ADAM6a et ADAM6b de souris expressibles insérés ; ou
dans lequel le génome comprend un gène ADAM6 de rat expressible inséré, chaque séquence d'acide nucléique d'ADAM6 étant inséré de sorte qu'il soit raccordé en service à un promoteur pour l'expression, et
dans lequel la composition est contenue dans un sachet intraveineux (IV) ou un conteneur raccordé à une seringue (IV).

2. Procédé selon la revendication 1, dans lequel un protocole amorce-rappel est utilisé pour l'immunisation ou dans lequel un protocole RIMMS est utilisé pour l'immunisation.

3. Procédé selon la revendication 1 ou la revendication 2, dans lequel le rongeur est une souris et dans lequel le génome de la souris comprend un locus de chaîne lourde d'anticorps transgénique sur une copie du chromosome 12.

4. Procédé selon la revendication 1 ou la revendication 2, dans lequel le rongeur est un rat et dans lequel le génome du rat comprend un locus de chaîne lourde d'anticorps transgénique sur une copie du chromosome 6.

5. Procédé selon la revendication 3 ou la revendication 4, dans lequel chaque gène ADAM6 inséré se trouve :
(i) sur un chromosome 12 lorsque l'animal est une souris ; ou
(ii) sur le chromosome 6 lorsque l'animal est un rat.

6. Procédé selon l'une quelconque des revendications précédentes, dans lequel un gène ADAM6 est inséré :
(i) dans l'un ou les deux loci de chaîne lourde transgéniques ou
(ii) dans les 20 Mb de l'un ou des deux loci de chaîne lourde transgéniques.

7. Procédé selon l'une quelconque des revendications précédentes, dans lequel les segments de gène humain remplacent tout ou partie de la région VDJ endogène dans chaque locus de chaîne lourde.

8. Procédé selon l'une quelconque des revendications précédentes, dans lequel le génome est homozygote pour chaque gène ADAM6 inséré ; éventuellement dans lequel le génome comprend plus de deux copies de gènes ADAM6 choisies parmi les gènes ADAM6 de rat, ADAM6a de souris et ADAM6b de souris.

9. Procédé selon l'une quelconque des revendications précédentes, dans lequel le génome comprend de l'ADAM6 expressible qui est inséré depuis un vecteur de ciblage, par exemple un vecteur BAC.

10. Procédé selon l'une quelconque des revendications précédentes, dans lequel le ou les gènes ADAM6 expressibles est ou sont une séquence nucléotidique d'ADAM6 de rat raccordée en service à un promoteur d'ADAM6 de souris endogène ou une séquence nucléotidique d'ADAM6 de souris raccordée en service à un promoteur d'ADAM6 de rat endogène.

11. Procédé selon l'une quelconque des revendications précédentes, dans lequel le génome comprend une région humaine VDJ insérée qui comprend des bases 105,400,051 à 106,368,585) issues du chromosome humain 14.

12. Procédé selon l'une quelconque des revendications précédentes, dans lequel le génome est homozygote sur la chaîne lourde d'Ig, sur les loci Igλ et Igκ.

13. Procédé selon l'une quelconque des revendications précédentes, dans lequel le génome comprend :
(a) des loci de chaînes lourdes, chacune comprenant un ou plusieurs segments de gène de chaîne lourde humaine V, un ou plusieurs segments de gène de chaîne lourde humaine D et un ou plusieurs segments de gène de chaîne lourde humaine JH en amont d'une région constante de souris ou de rat endogène ou une région humaine constante, par exemple Cmu et/ou Cgamma ;
(b) un locus de chaîne légère kappa éventuellement à l'état homozygote, comprenant un ou plusieurs segments de gène de chaîne humaine kappa V et un ou plusieurs segments de gène de chaîne humaine kappa Jκ en amont d'une région constante kappa de souris ou de rat endogène ; et éventuellement
(c) un locus de chaîne légère lambda éventuellement à l'état homozygote, comprenant un ou plusieurs segments de gène de chaîne humaine lambdaV et un ou plusieurs segments de gène de chaîne humaine lambda Jλ en amont d'une région constante lambda ; et
(d) dans lequel la souris ou le rat est capable de produire des anticorps chimériques après réaménagement desdits loci et immunisation avec un antigène.

14. Procédé selon l'une quelconque des revendications précédentes, dans lequel le rongeur est une souris et le génome comprend une insertion de VJ de chaîne humaine légère kappa ciblée dans le chromosome de souris 6 entre les coordonnées (70,673,899 et 70,675,515) commodément en position 70,674,734, ou dans une position équivalente dans le locus de souris lambda sur le chromosome 16.

15. Procédé selon l'une quelconque des revendications précédentes, dans lequel le VH humain qui est un VH naturellement inversé ou le pseudogène VH est omis du génome.


### SEQUENCE LISTING

<110> Kymab Limited
<120> TRANSGENIC ANIMALS
<130> 25077.20
<150> USSN 13/310,431
   <151> 2/12/2011
<150> GB1122047.2
   <151> 21/12/2011
<150> 13/416,684 <151> 09/03/2012
<160> 9
<170> PatentIn version 3.5
<210> 1
   <211> 2256
   <212> DNA
   <213> Rattus norvegicus
<400> 1
   atgttatctc tgacctgggg tatgaagcta gtggaaagat ctgtggtccc cagggtcctc 60
   ctcttgctct ttgcactctg gctgctcctc ctggttccag tccggtgttc tgaaggccac 120
   cccacttggc gctacatctc atcagaggtg gttattcctc ggaaggagat ctaccacagc 180
   aaaggaattc aaacacaagg acggctctcc tatagcttgc gttttagggg ccagagacat 240
   atcatccacc tgcgaagaaa gacactaatt tggcccagac acttgttgct gacaactcag 300
   gatgaccaag gagccttaca gatggattac ccttttttcc ctgtagattg ttactatttt 360
   ggctacctag agggaatccc tcaatccatg gtcactgtga atacttgcta tggaggcctg 420
   gaagggatca tgatgttgga tgaccttgcc tatgaaatca aacccctcaa cgattcacag 480
   gggtttgaac acattgtttc tcagatagta tcagagcctg atgtaacagg gcctacaaat 540
   acatggaaac gcttgaacct taatacaggt cctcccttat ccaggacaga gtatgccaat 600
   ggaactccca gaatgtctag taagaactac gcttcacatc cagctgctat aaaaggccaa 660
   ttccaagcaa ctaattctat atataaggaa agcaacaata ttgatactgc ggccaggtat 720
   ttgtttgagc tccttagtat aacggacagc tttctgatca ctattcatat gcggtactat 780
   gctattctct taactgtgtt taccgagagc gatccatttg cactagagta tacggtacca 840
   gggggctcta tttataacta ttatgtgtct aactttttta atcggttgag gcctgatgca 900
   tcaaccgtac ttaataaaga tgggccctcg gataacgact ttcatccagt tgaacagagt 960
   ttatgtactc ccgcaggcct gacgattgtt ggtcaacaca gacgaagttt tctagctcta 1020
   tctgttatga tcaccaatcg tattgcgatg tctttaggta taaaagctga tgatgagact 1080
   tactgcatct gccacagaag gaccacttgc attatgtaca aaaaccctga aataacagat 1140
   gctttcagca attgctccct tgtgcagata aaccagatac tgaatacccc tggtacaatg 1200
   tcatgccttt tctatgacca ccatgtttat cataatataa caaaaaccta caggttttgt 1260
   ggaaacttca agatagatat cggtgagcag tgtgactgtg gctcacataa ggcatgttac 1320
   gcagatccct gctgcggaag taattgcaag ttaactgctg gtagcatttg tgataaagaa 1380
   ttatgctgtg caaactgcac ctacagtcct tctgggacac tctgcagacc gatccagaac 1440
   atatgtgatc ttccagaata ctgtagtggg aataatatct tttgccctgc agacacttat 1500
   ctgcaagatg ggacgccatg ctcagaagag gggtactgct ataaaggcaa ctgcacagat 1560
   cgcagtgtgc agtgcaagga aatctttggt atgaatgcta agggtgctaa tatcaagtgc 1620
   tatgacatca acaaacaacg gtttcgattt gggcactgca ctagagcaca agagagcctc 1680
   atgtttaatg cttgctctga tcatgataaa ctgtgtggaa ggctgcagtg taccaacgtc 1740
   accaatcttc cattcctgca ggaacatgtt tcattccatc aatcggttat ctctgggttt 1800
   acctgctttg ggcttgatga acatcgtggg acagaaacaa cggatgctgg gctggtgaaa 1860
   catggtaccc cttgctccca aactaacttc tgcgatcgag gagcttgcaa tggaagttta 1920
   tctcggttgg attatgactg caccccagaa aaatgcaatt ttagaggagt gtgtaataat 1980
   catcggcatt gccattgtca tttaggttgg aaacctcctc tgtgcagaga ggaggggcct 2040
   agcgggagca cggacagtgg gtcccctccg aaggaaaggc gcacaataaa acagagcaga 2100
   gaaccactgt tatatttaag aatgctcttt ggtcgtcttt atttattcat tgtctcgctg 2160
   ctctttggag tggccactcg cgcaggagtt attaaggtct ttaagtttga agacttgcaa 2220
   gctgctctgc gggctgcaca agccaaggcg acttaa 2256
<210> 2
   <211> 2196
   <212> DNA
   <213> Oryctolagus cuniculus
<400> 2
   atggtgctgg cagaaggaca ggtcacgctg ctcctgcttg ggctctgggt gctcctagac 60
   ccaggtcagt gttccccagg ccgcccctcc tggcgctatg tctcatctga ggtggtgatt 120
   cctcggaagg agctgcacca gggcagaggt gttcaggtag caggctggct ctccatcagc 180
   ctgcactttg ggggccaaag acacgtcatc tgtatgcgga gcaagaagct tatttgggcc 240
   agacacctga tgatgatgac ccaagatgac caaggagcgt tgcagatgga ctatccttac 300
   attcctacag actgttacta cctcggccac ctggaagaca ttcctctgtc caccgtcacc 360
   attgacacgt gctatggggg cctggaaggc atcatgaagt tggatgacct cgcctatgaa 420
   atcaaacccc tcaaggactc caacacattt gaacacgttg tgtctcagat cgtggccgac 480
   cgcaatgcca cgggacccat gtacagactg gaacacgagg acgattttga ccccttcttc 540
   tccgaggtaa acagtagtgt ggctcccaag ctctctagtt tcaaccacat gtaccacatg 600
   gcccaattga aaggtcaaat tcaaatagcc cacgaaatgt atacggtact caacaatatt 660
   tcaaaatgca tccaattttc aataaacatg tttagtatta ttgacagttt tctgagagga 720
   attggcttta ggcactatat tgctctccta aacatataca accagcaaga gccagtcgtt 780
   atgaatgatt ttcgggttcc tggcggtcca atccatgctt attataaagc gaattttcat 840
   gacatctacc gcccttctcc atcgacattg attacaagaa atgcaccaaa tgatgattac 900
   caagaacccg ctaggtatgg cacctgtggc catcataact tgcttatcat tggttcccag 960
   ggcagacatt atctcctgtt ggctatttta actacacata aaattgcacg acagataggg 1020
   ttagcatatg actacagtgt ctgtgtgtgc cagagaagag caacctgctt gatgaggaaa 1080
   ttccctgaaa tgacagactc gttcagtaac tgctcttttg tccatacaca acatatagtt 1140
   tcaaacagat atatttttac atgctattac ttcacagata ggacgtacat gaataaaacc 1200
   ctgatacaga cgcgctgtgg aaacttttta gtggaagaaa gggagcaatg tgattgtggc 1260
   tccttcaagc attgttatgc caatgcatgc tgtcaaagcg actgtcgctt cacacctgga 1320
   agtatttgtg ataaacaaca atgctgcaca aactgcacct actcccccac ttcaaccctc 1380
   tgcagacctg tcatgaacat atgtgatctt ccagagtact gtggggggtc cacctacaca 1440
   tgccctgaaa atttttattt gcaagacgga accccgtgca ctgaagatgg ttactgctac 1500
   agagggaact gctctgaccc cactatgcac tgcaaggaga tctttggtca aagtgctgag 1560
   aatggtcctg cggattgcta tgccataaat ctcaacacct tccgatttgg acattgtaga 1620
   agagagcaac atcagaacgt ttaccatgct tgtgctgcac aagacaagga gtgtggaagg 1680
   ctacagtgca tcaatgtcac ccagcttcct cagttgcagg atcatgtttc attccatcag 1740
   tctgtgtaca atgagttcac ctgttttgga ctggatgaac accggtcaac aggatcaact 1800
   gatgctggac gtgtgagaga tggtactccc tgtggggaag gacttttctg tcttgagagc 1860
   agatgcaaca tgactatgct taacctgcat tacgactgtt tccctgagaa gtgcagtttt 1920
   agaggacttt gcaacaataa caagaattgc cactgccatg ttggctggga ccccccactg 1980
   tgcctgagtc cgggtgctgg tgggagctca caaagcgggc cccctccaag gagaatgcgc 2040
   acagtcacag atagcatgga gccaattctt tatttaagag tggtctttgc tcgtgtttat 2100
   tgttttattt ttgcactgct ctttggggta gccactaatg tgcgaacgat taagactacc 2160
   attgtccagg aacaaacagt taatgagcca cagtaa 2196
<210> 3
   <211> 2265
   <212> DNA
   <213> Mus musculus
<400> 3
   atgttatctc tgacctgggg catgaggcta gtggaaagac ctgtggtccc cagggtcctc      60
   ctcttgctat ttgcactctg gctgctcctc ctggttccag tctggtgttc tcaaggccat     120
   cccacttggc gttacatctc atcggaggtg gttattcctc ggaaggagat ctaccatacc     180
   aaaggacttc aagcacaaag actgctctcg tatagcttgc gttttcgggg ccagagacat     240
   atcatccacc tgcggagaaa gacactcatt tggcccagac acttgttgct gacaactcaa     300
   gatgaccaag gagccttaca gatggagtac cccttttttc ctgtagattg ttactatatt      360
   ggctacctgg aggggatcct gcaatccatg gtcactgtgg atacttgtta tgggggcctg     420
   tcaggggtca taaagttgga taaccttacc tatgaaatca aacccctcaa tgattcacag     480
   agctttgaac accttgtttc tcagatagta tctgagtctg atgacacagg gcctatgaat     540
   gcatggaagc actggagcca taatacaggt tctccctcct ccagattgga atatgcagat      600
   ggagctccca gactatctag taagaattac gctacacatc cagctgctat aaaaggccac      660
   tttcaagcaa cccattctgt atatagtgct tctggaggtg acaaactttc atctactgtt      720
   gagtatttgt ttaaagtcat tagtttaatg gacacctatc tgaccaatct tcatatgcgg      780
   tactatgtct ttctcatgac tgtgtatacc gaggctgatc cattttcaca agattttcga      840
   gttccaggag ggcaggctca tactttctat gagagagtat tttatgctca ttttaggcct      900
   gatgcaggag ctataattaa caagaattcg ccaggagatg atgctgttaa tccagctgag      960
   aggagtatat gttctccctc agccctaatt tgtcttggtc aacatggtcg aaatccttta     1020
   tttttatcta ttataataac caatcgtgtt ggaaggagtt taggcctaaa acatgatgag     1080
   gggtactgta tctgccagag aaggaacacc tgcatcatgt tcaaaaatcc tcaattaaca     1140
   gatgctttca gcaattgttc ccttgcagag ataagcaaca tacttaatac tcctgatctg     1200
   atgccatgtc ttttctatga ccgtcatgtt tattataata catcattgac ttataagttt     1260
   tgtggaaact tcaaagtaga taacaatgag cagtgtgact gtggctccca aaaggcatgt     1320
   tattcagatc cctgctgtgg aaatgattgc aggttaacac ctggtagcat ttgtgataaa     1380
   gaattatgct gtgcaaattg cacttacagt ccttctggga cactctgcag acctatccag     1440
   aacatatgtg atcttccaga gtactgtagt ggctctaagt tcatttgccc agatgacact     1500
   tatctgcaag atgggacacc atgctcagaa gagggttact gctataaagg taactgcact     1560
   gatcgcaaca tacaatgcat ggaaatcttt ggtgtaagtg ctaagaatgc taatattaag     1620
   tgctatgaca tcaacaaaca acggtttcga tttgggcatt gtactagagc agaagaaagc     1680
   ctcacattca atgcttgtgc tgatcaggac aagctgtgtg gaaggttgca gtgtaccaat     1740
   gtcaccaatc ttccattttt gcaagaacat gtttcattcc atcaatcggt tatctctggg     1800
   gttacctgct ttgggcttga tgaacatcgt gggacagaaa cagcagatgc tggattggtg     1860
   agacatggta ccccgtgttc aaggggtaag ttctgtgatc gaggagcttg caatggaagt     1920
   ttatctcgtt tgggttatga ctgcacccca gaaaaatgca atttcagagg agtgtgtaac     1980
   aatcgtcgga attgccattg ccattttggt tggagccctc caaagtgcaa agaagaggga     2040
   cacagtggga gcatagacag tgggtcccct ccggttcaaa ggcgcataat aaaacagaac     2100
   ctagagccag tagtgtattt aagaatactc tttggtcgta tttacttcct ctttgttgca     2160
   ctgctctttg gcattgccac tcgtgtagga gttactaaga tatttaggtt tgaagacttg     2220
   caagctgctt tacgttcttg gcaagaacaa gcaaaggaca agtaa                     2265
<210> 4
   <211> 2271
   <212> DNA
   <213> Mus musculus
<400> 4
   atgttatctc tgacctgggg catgaggcta gtggaaagac ctgtggtccc cagggtcctc 60
   ctcttgctat ttgcactctg gctgctcctc ctggttccag tctggtgttc tcaaggccat 120
   cctacttggc gttacatctc atcggaggtg gttattcctc ggaaggagat ctaccatacc 180
   aaaggacttc aagcacaaag actgctctca tatagcttgc attttcgggg ccagagacat 240
   atcatccacc tgcggagaaa gacactcatt tggcccagac acttgttgct gacaactcaa 300
   gatgaccaag gagccttaca gatggattac cccttttttc ctgtagattg ttactatatt 360
   ggctacctgg aggggatccc acaatccatg gtcactgtgg atacttgtta tgggggcctg 420
   tcaggggtca tgaagttaga tgaccttacc tatgaaatca aacccctcaa tgattcacag 480
   agctttgaac accttgtttc tcagatagta tctgagtctg atgacacagg gcctatgaat 540
   gcatggaagc actggagcca taatacaggt tctccctcct ccagattgga atatgcagat 600
   ggagctccca gaatatctag taagaactac gctacacatc cagctgctat aaaaggccac 660
   tttcaagcaa ccaattctgt atataattct gctgcaggtg acaaactttc atctactgtt 720
   gggtatttgt ttcaagtcat tagtttaatg gacacctatc tgaccaatct tcatatgcgg 780
   tactatgtct ttctcatgac tgtgtacacc aattctgatc catttcgact tgagtttgca 840
   gttccaggag ggtcggctta taattactat gtgtcagtct tttataataa atttaagcct 900
   gatgcaggag ttttacttaa taagtatggg ccacaagata accaggttaa tccagctgag 960
   aggagtatat gttcttcctt agccctaatt tgtattggta aatatgatcg aaatccttta 1020
   tttttatctc ctataataac caatcgtgtt ggaaggagtt taggcttaaa atatgatgag 1080
   gggtactgtg tctgccagag aaggaacacc tgcattatgt tcagacatcc tcaattaaca 1140
   gatgctttca gcaattgttc ccttgcagag ataagcaaca tacttaatac tcctggtctg 1200
   atgccatgtc ttttctatga ccgtcatgtt tattataata catcattgac ttataagttt 1260
   tgtggaaact tcaaagtaga taacgatgag cagtgtgact gtggctccca aaaggcatgt 1320
   tattcagatc cctgctgtgg aaatgattgc aggttaacac ctggtagcat ttgtgataaa 1380
   gaattatgct gtgcaaattg cacttacagt ccttctggga cactctgcag acctatccag 1440
   aacatatgtg atcttccaga gtactgtaat gggactaaat acatttgccc agatgacact 1500
   tatctgcaag atgggacacc atgctcagaa gatggttact gctataaagg taactgcact 1560
   gatcgcaaca tacaatgcat ggaaatcttt ggtgtaagtg ctaagaatgc taatattaag 1620
   tgctatgaca tcaacaaaca acggtttcga tttgggcatt gtactagagc agaagaaagc 1680
   ctcacattca atgcttgtgc tgatcaggac aagctgtgtg gaaggttgca gtgtaccaat 1740
   gtcaccaatc ttccatattt gcaagaacat gtttcattcc atcaatcgat tatctctggg 1800
   tttacctgct ttgggcttga tgaacatcgt gggacagaaa caacagatgc tggaatggtg 1860
   agacatggta ccccctgctc caaaagtaag ttctgtgatc aaggagcttg cagtggaagt 1920
   ttatctcatt tgggttatga ctgcacccca gaaaaatgca gttttagagg agtgtgtaac 1980
   aatcatcgga attgccattg tcattttggt tggaagcctc cagagtgcaa agaagaggga 2040
   ctaagtggga gcatagacag tgggtcccct ccagttcaaa ggcacacaat aaaacaaaaa 2100
   caagagccag tggtgtattt aagaatactc tttggtcgta tttacttcct ctttgttgca 2160
   ctgctctttg gcattgccac tcgtgtagga gttactaaga tttttagatt tgaagacttg 2220
   caagctactt tacgttctgg gcaaggacca gcaagggaca agccaaagta a 2271
<210> 5
   <211> 200
   <212> DNA
   <213> Artificial sequence
<220>
   <223> 5' Homology Arm:
<400> 5
   tatgttgatg gatttccata tattaaacca tccctgcatc cctgggatga agcctacttg      60
   gtcatgatag acgattgttt tgatgtgttc ttggattcag ttagtgagaa atatattgag     120
   tatttttaca tcgatattca taagggaaat tggtctgaag ttctctttct ttgttgggtc     180
   tttatgtggt ttagttatca                                                 200
<210> 6
   <211> 204
   <212> DNA
   <213> Artificial Sequence
<220>
   <223> 3' Homology Arm:
<400> 6
   tgattccacc agaggttctt ttatccttga gaagagtttt tgctatccta ggttttttgt       60
   tattccacat gaatttgcag attgctcttt ctaattcctt gaagaattga gttggaattt     120
   gatggggatt gcattaaatc tgtagattcc ttttggcaag acagccattt ttacaatgtt     180
   aatcctgcca atccatgagc atgg                                            204
<210> 7
   <211> 304
   <212> DNA
   <213> Artificial sequence
<220>
   <223> 5' Homology Arm:
<400> 7
   tttatgtact ataccatctc agaaagtcag gttagtctca ctagcatcgt aaaagctctg      60
   tctgggcttt tccatctgct ctgctttttg tctctgtgtc taaaaatata taaaccaatg     120
   ttgtccagcc aaaaaaaaaa aattaaagag caaaaggagg taaaatggat acaaattgga     180
   aaagaagaaa tcaaaatatc actacttgaa gatagtataa tatatttaac tgaccacaaa     240
   aattccacca gaaaactcct aaacctgata aacaaactca gaaaaatggc tagatataaa     300
   ctta                                                                  304
<210> 8
   <211> 315
   <212> DNA
   <213> Artificial Sequence
<220>
   <223> 3' Homology Arm:
<400> 8
   acccatagag agaaaacagg tgagttagtg cattaaaggg gctgagcagg gagttctcat    60
   cgctccccag caccagaaat aagagcctct ccggagctgc tgggacatgg aatgcagatg   120
   attcggacca tcagccccac agagaccttt cccactctgg ctcagaaaga ggcactggac   180
   cacagttgga gaggagaatc gaaagctgat atctctgtat tcacttagcc tgttacccac   240
   ccatgcaccc aagtccaagg tgggagaaac actgagggtc taaacacagc cccagagcaa   300
   ctgccagtat taaat                                                    315
<210> 9
   <211> 365
   <212> DNA
   <213> Artificial Sequence
<220>
   <223> IgH BAC Homology Arm:
<400> 9
   attcaggcag ttaattgttg ggttcatgtt ttacaactaa agaataaatt caggccagat    60
   gcagtggatc atcgctataa tcacaccact ttcagaagca aaaatgaggg aaatcccgtg   120
   agacgaggca atcgaagcca acctgagcaa cataaagaga tgctatttct ctgaaaaaat   180
   attttaaaga ataagcaggt gaggggtggc gttcccctct acttctagat actcaggaag   240
   caaagatggg aagattatgt gagccaggtg ttcaaaatta cagtgagctt tgatcataca   300
   actgttcttc aaactgtgca acagggtgag agcctgtctc taaaaacaaa taaaaaagaa   360
   tcaat                                                               365